# EUROPEAN PATENT APPLICATION

(11) **EP 4 477 249 A1**
(43) Date of publication of application: **18.12.2024**
(21) Application number: 23834587.0
(22) Date of filing: 08.06.2023
(51) Int. Cl.: A61M 60/122

(54) **DRIVING MECHANISM AND BLOOD PUMP**

(30) Priority: 08.07.2022 CN 202210800376; 15.08.2022 CN 202210977269
(71) Applicant: Shenzhen Core Medical Technology Co., Ltd., Shenzhen, Guangdong 518000 (CN)
(72) Inventor: XIE, Duanqing, Shenzhen, Guangdong 518000 (CN); YU, Shunzhou, Shenzhen, Guangdong 518000 (CN)
(74) Representative: Appleyard Lees IP LLP
(86) International application number: PCT/CN2023/099070
(87) International publication number: WO 2024/007813

(57) **Abstract**

Provided are a driving mechanism (10) and a blood pump (1). The driving mechanism (10) comprises a housing (100), a rotating shaft (200), a rotor (400), a first shaft sleeve (510), a second shaft sleeve (520), and a stopping member (600). The rotating shaft (200) is rotatably mounted on the housing (100). The rotating shaft (200) has a connecting end (210) and a ball head end (220) away from the connecting end (210). The connecting end (210) is configured to be connected to an impeller (20). The rotor (400) is fixedly connected to the rotating shaft (200). The first shaft sleeve (510) is provided with a groove (512). The rotating shaft (200) is rotatably arranged on the second shaft sleeve (520) in a penetrating manner. The ball head end (220) is movably arranged in the groove (512). The stopping member (600) is fixedly connected to at least one of the rotating shaft (200) and the rotor (400). The stopping member (600) can abut against the second shaft sleeve (520).

## Description

### CROSS-REFERENCE TO RELATED APPLICATIONS

This application claims priority to Chinese Patent Application No. CN202210800376.5, filed on July 8, 2022, and Chinese Patent Application No. CN202210977269.X, filed on August 15, 2022. The contents of the above identified applications are hereby incorporated herein in their entireties by reference.

### TECHNICAL FIELD

The present application relates to the technical field of medical devices, and in particular to a driving mechanism and a blood pump.

### BACKGROUND

Blood pump is designed to be inserted percutaneously into a patient's blood vessel, such as an artery or a vein in a thigh or an axilla, and can be advanced into the patient's heart to function as a left ventricular assist device or a right ventricular assist device. Therefore, the blood pump may also be referred to as an intracardiac blood pump or an intravascular blood pump.

The blood pump generally has a driving mechanism and an impeller, and the impeller is connected to a driving shaft of the driving mechanism. In order to achieve stable rotation of the driving shaft, a structure for positioning or limiting the driving shaft is usually required, which makes the structure of the driving mechanism relatively complicated.

### SUMMARY

Accordingly, the present application provides a driving mechanism and a blood pump having a relatively simple structure.

An embodiment of a first aspect of the present application provides a driving mechanism, which includes a housing, a rotating shaft, a rotor, a first shaft sleeve, and a second shaft sleeve. The rotating shaft is rotatably mounted to the housing, the rotating shaft includes a connecting end configured to be connected to the impeller and a spherical head end away from the connecting end. The rotor is fixedly connected to the rotating shaft. Both the first shaft sleeve and the second shaft sleeve are mounted to the housing, the first shaft sleeve is provided with a groove having a concave spherical wall, the rotating shaft rotatably extends through the second shaft sleeve, the spherical head end is movably provided in the groove and is capable of abutting against the spherical wall, and the rotor is located between the first shaft sleeve and the second shaft sleeve. The blocking member is fixedly connected to at least one of the rotating shaft and the rotor. The blocking member is located between the rotor and the second shaft sleeve, and the blocking member is capable of abutting against the second shaft sleeve.

An embodiment of a second aspect of the present application provides a blood pump, which includes an impeller and the driving mechanism as described in the first aspect. The impeller is connected to the connecting end of the rotating shaft and is capable of rotating along with the rotating shaft.

Details of one or more embodiments of the present application are set forth in the following drawings and descriptions. Other features, objects and advantages of the present application will become apparent from the description, drawings, and claims.

### BRIEF DESCRIPTION OF THE DRAWINGS

To illustrate the technical solutions in the embodiments of the present application or in the prior art more clearly, the accompanying drawings for describing the embodiments or the prior art are introduced briefly in the following. Apparently, the accompanying drawings in the following description are only some embodiments of the present application, and persons of ordinary skill in the art can derive other drawings from the accompanying drawings without creative efforts.
FIG. 1 is a perspective view of a blood pump according to an embodiment of the present application.
FIG. 2 is a cross-sectional view of the blood pump shown in FIG. 1 with a cannula, an impeller, and a part of a catheter omitted.
FIG. 3 is a partial enlarged view of the blood pump shown in FIG. 2.
FIG. 4 is a cross-sectional view of a shaft, a blocking member, a rotor, a first shaft sleeve, and a second shaft sleeve of FIG. 1 assembled together.
FIG. 5 is an enlarged view of a portion A shown in FIG. 4.
FIG. 6 is a perspective view of a rotating shaft of the blood pump shown in FIG. 1.
FIG. 7 is a perspective view of a rotor, a stator, and a magnetic conduction member of the blood pump shown in FIG. 1 assembled together.
FIG. 8 is a perspective view of a second rotor and a magnetic conduction plate of the magnetic conduction member shown in FIG7 assembled together.
FIG. 9 is a perspective view of a first flywheel of a first rotor of the rotor shown in FIG. 7.
FIG. 10 is an enlarged view of a portion B shown in FIG. 2.
FIG. 11 is a perspective view of the first shaft sleeve of the blood pump shown in FIG. 2.
FIG. 12 is a cross-sectional view of the second shaft sleeve of FIG. 11.
FIG. 13 is a cross-sectional view of the blood pump shown in FIG. 1 viewed from another aspect with a cannula, an impeller, and a part of a catheter omitted.
FIG. 14 is an enlarged view of a portion C of FIG. 13.
FIG. 15 is a perspective view of a supporting base of the blood pump shown in FIG. 13.
FIG. 16 is a perspective view of a second shaft sleeve of the blood pump shown in FIG. 2.
FIG. 17 is a perspective view of a blood pump according to an embodiment of the present application.
FIG. 18 is an exploded view of the blood pump of FIG. 17.
FIG. 19 is a perspective view of a cannula assembly of the blood pump of FIG. 17.
FIG. 20 is an enlarged view of a portion D in FIG. 19.
FIG. 21 is a perspective view of a connecting tube of the cannula assembly of FIG. 19.
FIG. 22 is a schematic view of an internal structure of the connecting tube of FIG. 21.
FIG. 23 is a schematic view of an internal structure of the connecting tube of FIG. 21.
FIG. 24 is an exploded view of the blood pump of FIG. 17 with an inserting tube and the connecting tube omitted.
FIG. 25 is another exploded view of the blood pump of FIG. 17 with the inserting tube and the connecting tube omitted.
FIG. 26 is a cross-sectional view of the blood pump of FIG. 17 with the inserting tube, the connecting tube, and a portion of the catheter omitted.
FIG. 27 is an enlarged view of a portion E of FIG. 26.
FIG. 28 is a schematic view of a rotating shaft, a first shaft sleeve and a second shaft sleeve in FIG. 26 assembled together.
FIG. 29 is an assembly schematic view of the first shaft sleeve and a portion of the rotating shaft of FIG. 28.
FIG. 30 is a schematic view of a positional relationship between the second shaft sleeve and the rotating shaft in FIG. 28 when the rotating shaft is deflected.
FIG. 31 is a cross-sectional view of the first shaft sleeve of FIG. 26.
FIG. 32 is a perspective view of a supporting base of FIG. 26.
FIG. 33 is a perspective view of the second shaft sleeve of FIG. 26.
FIG. 34 is a perspective view of a first rotor of FIG. 26.
FIG. 35 is a perspective view of a second rotor of FIG. 26.
FIG. 36 is an exploded view of a stator and a magnetic conduction member of FIG. 26.

### DETAILED DESCRIPTION OF THE EMBODIMENTS

In order to make the objectives, technical solutions, and advantages of the present application more clearly understood, the present application is described in further detail below in conjunction with the accompanying drawings and embodiments. It should be understood that specific embodiments described herein are intended only to interpret the present application and not intended to limit the present application.

It should be noted that when one element is referred to as "fixed to" or "arranged on" another element, it may be directly disposed on the other element or an intermediate element may exist. When one element is considered to be "connected to" another element, it may be directly connected to another element or indirectly connected to another element.

In addition, the terms "first" and "second" are used for descriptive purposes only, which cannot be construed as indicating or implying a relative importance, or implicitly specifying the number of the indicated technical features. Thus, the features defined with "first" and "second" may explicitly or implicitly include one or more features. In the description of the present application, "a plurality of' means two or more, unless specifically stated otherwise.

In order to illustrate the technical solutions of the present application, description will be made below with reference to specific drawings and embodiments.

In the field of interventional medicine, an end of a device adjacent to an operator is usually defined as a proximal end, and an end of the device away from the operator is defined as a distal end.

A driving mechanism 10 and a blood pump 1 according to an embodiment of the present application are now described.

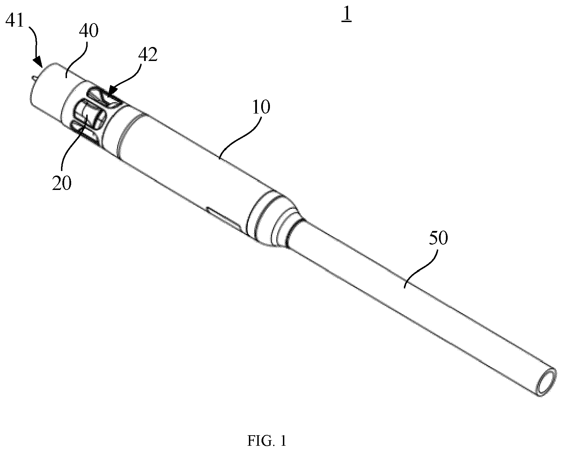

Referring to FIG. 1, the blood pump 1 includes a driving mechanism 10 and an impeller 20. The driving mechanism 10 is in transmission connection with the impeller 20, and the driving mechanism 10 is capable of driving the impeller 20 to rotate. Specifically, the blood pump 1 further includes a cannula 40 fixedly connected to a distal end of the driving mechanism 10. The impeller 20 is rotatably accommodated in the cannula 40. The cannula 40 has a blood outlet 41 and a blood inlet 42. When the impeller 20 rotates, blood flows from the blood inlet 42 into the cannula 40 and then flows out of the blood outlet 41. In an embodiment, the cannula 40 extends through a heart valve, such as an aortic valve. The blood inlet 42 is located within a heart, and the blood outlet 41 and the driving mechanism 10 are located in a blood vessel, such as an aorta outside a heart. Specifically, the blood pump 1 further includes a catheter 50 connected to a proximal end of the driving mechanism 10. The catheter 50 is configured to accommodate various supply lines. For example, the supply lines include a wire configured to be electrically connected to the driving mechanism 10 and a cleaning pipeline configured to supply flushing fluid to the blood pump 1. Optionally, the flushing fluid is physiological saline, physiological saline containing heparin, or glucose, etc.

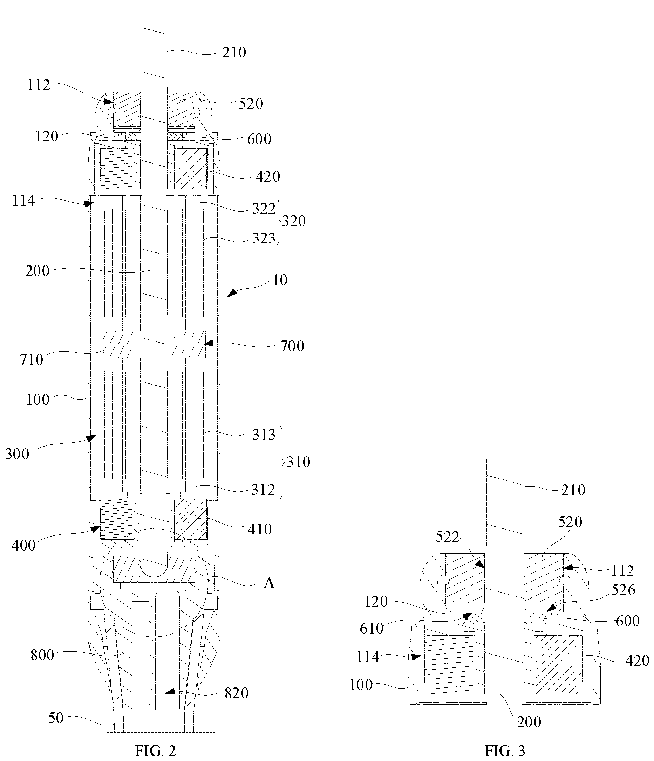

Referring to FIG. 2 to FIG. 6, the driving mechanism 10 includes a housing 100, a rotating shaft 200, a stator 300, a rotor 400, a first shaft sleeve 510, a second shaft sleeve 520, and a blocking member 600. The housing 100 is substantially a cylindrical housing with openings at both ends thereof. A distal end of the housing 100 is fixedly connected to the cannula 40, and a proximal end of the housing 100 is fixedly connected to the catheter 50. The housing 100 has an inner cavity. Specifically, a separating ring 120 is provided in the housing 100, and the separating ring 120 divides the inner cavity of the housing 100 into a limiting cavity 112 and an accommodating cavity 114. In the illustrated embodiment, the limiting cavity 112 and the accommodating cavity 114 are arranged along an axial direction of the housing 100.

The rotating shaft 200 is elongated. The rotating shaft 200 is rotatably mounted to the housing 100, and includes a connecting end 210 configured to be connected to the impeller 20 and a spherical head end 220 away from the connecting end 210. In the illustrated embodiment, the rotating shaft 200 extends substantially along the axial direction of the housing 100, in other words, an axis of the rotating shaft 200 extends in a direction substantially consistent with the axial direction of the housing 100. The limiting cavity 112 and the accommodating cavity 114 are arranged along the axis of the rotating shaft 200. The rotating shaft 200 extends through the limiting cavity 112, is partially accommodated in the accommodating cavity 114, and is partially located outside the housing 100 or extends partially into the cannula 10. A portion of the rotating shaft 200 extending out of the housing 100 or extending into the cannula 10 is the connecting end 210 of the rotating shaft 200. The spherical head end 220 is located in the accommodating cavity 114. Specifically, the impeller 20 is fixedly connected to the connecting end 210, so that the impeller 20 can rotate along with the rotating shaft 200. In some embodiments, the rotating shaft 200 is made of a ceramic material. Compared with metal materials, ceramics have higher processing precision, higher biocompatibility and mechanical strength, and better wear resistance and corrosion resistance.

The stator 300 is fixedly mounted to the housing 100, that is, the stator 300 is provided in an inner cavity of the housing 100. In the illustrated embodiment, the stator 300 is located in the accommodating cavity 114. The rotating shaft 200 rotatably extends through the stator 300. The rotor 400 is located in the housing 100, i.e., the rotor 400 is also provided in the inner cavity of the housing 100. In the illustrated embodiment, the rotor 400 is located in the accommodating cavity 114. The rotor 400 is fixedly connected to the rotating shaft 200, the stator 300 can drive the rotor 400 to rotate, and the rotor 400 can drive the rotating shaft 200 to rotate. Specifically, the rotor 400 is magnetic, and the stator 300 is capable of generating a rotating magnetic field that drives the rotor 400 to rotate.

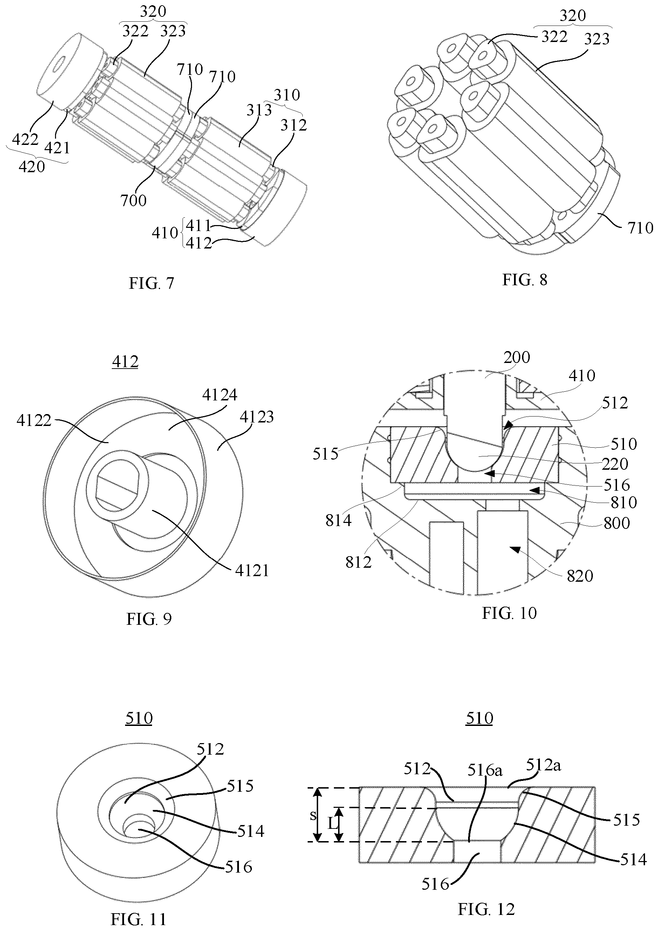

Referring to FIG. 7, in the illustrated embodiment, the rotor 400 includes a first rotor 410 and a second rotor 420, and both the first rotor 410 and the second rotor 420 are fixedly connected to the rotating shaft 200. Both the first rotor 410 and the second rotor 420 are rotatably accommodated in the accommodating cavity 114 of the housing 100. The first rotor 420 and the second rotor 420 are arranged along the axis of the rotating shaft 200. The stator 300 is located between the first rotor 410 and the second rotor 420. Both the first rotor 410 and the second rotor 420 are magnetic, and the stator 300 is capable of generating a rotating magnetic field that drives the first rotor 410 and the second rotor 420 to rotate. Specifically, the first rotor 410 includes a first magnet 411 fixedly connected to the rotating shaft 200. The first magnet 411 is an annular Halbach array magnet. Specifically, the first rotor 410 further includes a first flywheel 412 fixedly connected to the rotating shaft 200, and the first magnet 411 is fixedly connected to the first flywheel 412. By providing the first flywheel 412, the connection strength between the first magnet 411 and the rotating shaft 200 can be enhanced. In addition, the shaking of the rotating shaft 200 during rotation can be reduced, so that the whole rotating shaft 200 is more stable during rotation.

Referring to FIG. 9, specifically, the first flywheel 412 includes a first inner tube 4121, a first disc-shaped portion 4122, and a first outer annular wall 4123. Both the first inner tube 4121 and the first outer annular wall 4123 are of circular tubular structures, and the first disc-shaped portion 4122 is of an annular disc structure. Both the first inner tube 4121 and the first outer annular wall 4123 are fixedly connected to the first disc-shaped portion 4122. The first outer annular wall 4123 surrounds the first disc-shaped portion 4122, the first inner tube 4121 and the first outer annular wall 4123 are arranged coaxially, and the rotating shaft 200 extends through the first inner tube 4121 and is fixedly connected to the first inner tube 4121. A first annular cavity 4124 is formed between the first inner tube 4121 and the first outer annular wall 4123. The first magnet 411 is accommodated in the first annular cavity 4124. A shape of the first annular cavity 4124 is adapted to the first magnet 411, so as to facilitate mounting and positioning the first magnet 411. Such arrangement enables the first flywheel 412 to limit the first magnet 411, which not only facilitates the mounting of the first magnet 411, but also enables the first magnet 411 and the first flywheel 412 to be combined more stably.

It should be noted that the first flywheel 412 is not limited to the above structure. In some embodiments, the first flywheel 412 does not have the first outer annular wall 4123. In some embodiments, the first flywheel 412 does not have the first outer annular wall 4123 and the first inner tube 4121, and at this time, the rotating shaft 200 fixedly extends through a center of the first disc-shaped portion 4122. Compared to the first flywheel 412 having only the first disc-shaped portion 4122, the first inner tube 4121 enables the first flywheel 412 to be more stably connected to the rotating shaft 200. The second rotor 420 includes a second magnet 421 fixedly connected to the rotating shaft 200. Specifically, the second magnet 421 is an annular Halbach array magnet. Specifically, the second rotor 420 further includes a second flywheel 422 fixedly connected to the rotating shaft 200, and the second magnet 421 is fixed to the second flywheel 422. By providing the second flywheel 422, the connection strength between the second magnet 421 and the rotating shaft 200 can be enhanced. In addition, the shaking of the rotating shaft 200 during rotation can be reduced, so that the whole rotating shaft 200 is more stable during rotation.

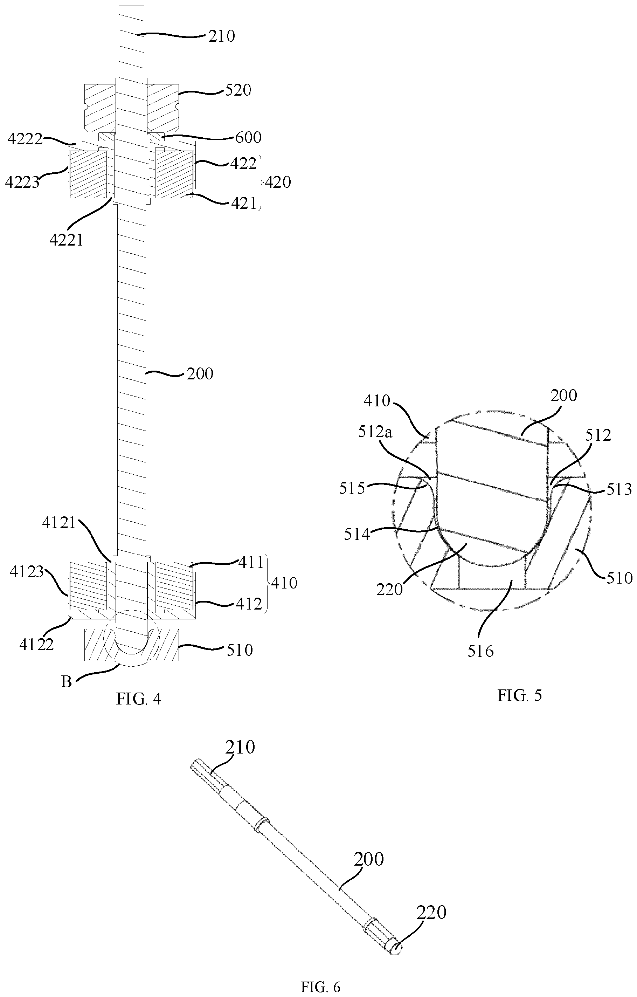

Specifically, referring to FIG. 4, the second flywheel 422 includes a second inner tube 4221, a second disc-shaped portion 4222, and a second outer annular wall 4223. Both the second inner tube 4221 and the second outer annular wall 4223 are of circular tubular structures, and the second disc-shaped portion 4222 is of an annular disc structure. Both the second inner tube 4221 and the second outer annular wall 4223 are fixedly connected to the second disc-shaped portion 4222. The second outer annular wall 4223 surrounds the second disc-shaped portion 4222, the second inner tube 4221 and the second outer annular wall 4223 are arranged coaxially, and the rotating shaft 200 extends through the second inner tube 4221 and is fixedly connected to the second inner tube 4221. A second annular cavity is formed between the second inner tube 4221 and the second outer annular wall 4223. The second magnet 421 is accommodated in the second annular cavity. A shape of the second annular cavity is adapted to the second magnet 421, so as to facilitate mounting and positioning of the second magnet 421. Such arrangement enables the second flywheel 422 to limit the second magnet 421, which not only facilitates the mounting of the second magnet 421, but also enables the second magnet 421 and the second flywheel 422 to be combined more stably.

It should be noted that the second flywheel 422 is not limited to the above structure. In some embodiments, the second flywheel 422 does not have the second outer annular wall 4223. In some embodiments, the second flywheel 422 does not have the second outer annular wall 4223 and the second inner tube 4221, and at this time, the rotating shaft 200 fixedly extends through a center of the second disc-shaped portion 4222. Compared to the second flywheel 422 having only the second disc-shaped portion 4222, the second inner tube 4221 enables the second flywheel 422 to be more stably connected to the rotating shaft 200.

Specifically, the stator 300 includes a first stator unit 310 and a second stator unit 320 arranged along the axis of the rotating shaft 200. The first stator unit 310 is capable of driving the first rotor 410 to rotate, and the second stator unit 320 is capable of driving the second rotor 420 to rotate. Specifically, the first stator unit 310 can generate a rotating magnetic field to drive the first rotor 410 to rotate, and the second stator unit 320 can generate a rotating magnetic field to drive the second rotor 420 to rotate. Both the first stator unit 310 and the second stator unit 320 are fixedly accommodated in the accommodating cavity 114 of the housing 100. The rotating shaft 200 rotatably extends through the first stator unit 310 and the second stator unit 320. The first stator unit 310 and the second stator unit 320 are both located between the first rotor 410 and the second rotor 420.

The first stator assembly 310 and the second stator assembly 320 each includes a magnetic core and a coil wound around the magnetic core. Specifically, the first stator assembly 310 includes a first magnetic core 312 and a first coil 313 wound around the first magnetic core 312. A plurality of first magnetic cores 312 are provided, and the plurality of first magnetic cores 312 are arranged around the axis of the rotating shaft 200 for one circumference. Each first magnetic core 312 corresponds to one first coil 313. The structure of the second stator assembly 320 is similar to that of the first stator assembly 310. Referring to FIG. 8, the second stator assembly 320 includes a second magnetic core 322 and a second coil 323 wound around the second magnetic core 322. A plurality of second magnetic cores 322 are provided, and the plurality of second magnetic cores 322 are arranged around the axis of the rotating shaft 200 for one circumference. Each second magnetic core 322 corresponds to one second coil 323.

Specifically, the driving mechanism 10 further includes a magnetic conduction member 700 fixedly connected to the housing 100. Both the first magnetic core 312 of the first stator unit 310 and the second magnetic core 322 of the second stator unit 320 are fixedly connected to the magnetic conduction member 700. Specifically, the magnetic conduction member 700 is fixedly accommodated in the housing 100, e.g., snapped into an inner side wall of the housing 100. e.g., snapped into an inner side wall of the housing 100. The rotating shaft 200 rotatably extends through the magnetic conduction member 700. One end of the first magnetic core 312 is fixedly connected to the magnetic conduction member 700, and the first rotor 410 is adjacent to the other end of the first magnetic core 312. One end of the second magnetic core 322 is fixedly connected to the magnetic conduction member 700, and the second rotor 420 is adjacent to the other end of the second magnetic core 322.

The magnetic conduction member 700 serves to close a magnetic circuit, so as to promote and increase generation of the magnetic flux and improve a coupling capability. Therefore, the magnetic conduction member 700 can function to close the magnetic circuit between the first stator assembly 310 and the first rotor 410, and to close the magnetic circuit between the second stator assembly 320 and the second rotor 420, so as to increase the magnetic flux. Therefore, the arrangement of the magnetic conduction member 700 helps to reduce an overall diameter of the driving device 10. In addition, both the first magnetic core 312 of the first stator assembly 310 and the second magnetic core 322 of the second stator assembly 320 are fixedly connected to the magnetic conduction member 700, so that the first stator assembly 310 and the second stator assembly 320 can be positioned and mounted, and the assembly difficulty of the first stator assembly 310 and the second stator assembly 320 is reduced. At the same time, the magnetic conduction member 700 provided in the above manner can also reduce arrangement of a positioning structure in the housing 100, thereby simplifying the structure of the housing 100 and simplifying the assembly process of the entire driving device 10.

Specifically, the magnetic conduction member 700 includes two magnetic conduction plates 710 that are stacked. One magnetic conduction plate 710 is fixedly connected to the first magnetic core 312 of the first stator assembly 310, and the other magnetic conduction plate 710 is fixedly connected to the second magnetic core 322 of the second stator assembly 320. The rotating shaft 200 rotatably extends through the two magnetic conduction plates 710. Specifically, the two magnetic conduction plates 710 are separated prior to assembly. Since the magnetic conduction member 700 is configured as the two magnetic conduction plates 710 that are separated prior to assembly, when assembling the driving device 10, the first magnetic core 312 can be first fixedly connected to the magnetic conduction plate 710, the second magnetic core 322 can be fixedly connected to the other magnetic conduction plate 710, and then the two magnetic conduction plates 710 can be stacked. In this way, the first magnetic core 312 and the second magnetic core 322 can be easily assembled to the two magnetic conduction plates 710, respectively, and the first magnetic core 312 and the second magnetic core 322 can be assembled more easily.

Specifically, the two magnetic conduction plates 710 are fixedly connected, so that the first stator assembly 310, the second stator assembly 320, and the magnetic conduction member 700 are integrally formed and assembled into the housing 100, and the assembly of the stator 300 is easier. For example, the two magnetic conduction plates 710 may be connected together by gluing or welding. It should be understood that in other embodiments, the two magnetic conduction plates 710 are not fixedly connected, but are in contact with each other. It should be noted that the magnetic conduction member 700 is not limited to the combination of the two magnetic conduction plates 710 that are separated, and the magnetic conduction member 700 may be a plate-like structure. Both the first magnetic core 312 and the second magnetic core 322 are connected to the magnetic conduction member 700, that is, the first stator assembly 310 and the second stator assembly 320 share one magnetic conduction member 700.

Specifically, the magnetic conduction plate 710 is made of silicon steel, and the first magnetic core 312 and the second magnetic core 322 are made of silicon steel.

Referring to FIG. 2, FIG. 4 and FIG. 5 again, both the first shaft sleeve 510 and the second shaft sleeve 520 are both mounted on the housing 100. Specifically, the first shaft sleeve 510 is accommodated in the accommodating cavity 114, and the second shaft sleeve 520 is accommodated in the limiting cavity 112. Both the first shaft sleeve 510 and the second shaft sleeve 520 are fixedly connected to the housing 100. The first shaft sleeve 510 and the second shaft sleeve 520 are spaced apart from each other along the axial direction of the housing 100. The first shaft sleeve 510 and the second shaft sleeve 520 can limit the rotating shaft 200. The second shaft sleeve 520 is provided closer to the connecting end 210 of the rotating shaft 200 than the first shaft sleeve 510. In other words, the second shaft sleeve 520 is provided closer to the separating ring 120 in the housing 100 than the first shaft sleeve 510. The rotor 400 is located between the first shaft sleeve 510 and the second shaft sleeve 520. The stator 300 is also located between the first shaft sleeve 510 and the second shaft sleeve 520. In the illustrated embodiment, the first rotor 410, the second rotor 420, the first stator unit 310, and the second stator unit 320 are located between the first shaft sleeve 510 and the second shaft sleeve 520. The first rotor 410 is adjacent to the first shaft sleeve 510, and the second rotor 420 is adjacent to the second shaft sleeve 520. In other words, the first shaft sleeve 510, the first rotor 410, the first stator unit 310, the second stator unit 320, the second rotor 420, and the second shaft sleeve 520 are arranged sequentially along the axis of the rotating shaft 200, and the second shaft sleeve 510 is located closest to the connecting end 210 of the rotating shaft 200.

The first shaft sleeve 510 is provided with a groove 512 having a concave spherical wall 514. The rotating shaft 200 rotatably extends through the second shaft sleeve 520. The spherical head end 220 of the rotating shaft 200 is movably provided in the groove 512 and can abut against the spherical wall 514. The groove 512 can support and limit the spherical head end 220 of the rotating shaft 200, so as to limit a movement range of the rotating shaft 200 along the axis of the rotating shaft 200 in a direction away from the impeller 20, and at the same time, to limit a swinging range of the spherical head end 220 of the rotating shaft 200 in a radial direction of the rotating shaft 200.

Referring to FIG. 10, FIG. 11 and FIG. 12, specifically, the groove 512 has a notch 512a, and the rotating shaft 200 extends through the notch 512a. The notch 512a of the groove 512 is rounded 515, i.e., a groove wall at the notch 512a of the groove 512 is rounded, so as to prevent the rotating shaft 200 from being scratched and worn by the notch 512a of the groove 512 having sharp corners.

Specifically, a length h of the spherical head end 220 in an axial direction of the rotating shaft 200 is less than a depth s of the groove 512 (the depth s of the groove 512 is the maximum distance between the notch 512a of the groove 512 and the spherical wall 514), so as to better limit the spherical head end 220 in the groove 512, and reduce a radial swinging range of the rotating shaft 200. Meanwhile, the depth of the groove 512 should not be too large to prevent a length of the rotating shaft 200 in the groove 512 from being too long, otherwise the length of the rotating shaft 200 may be increased. In the illustrated embodiment, a radius of the spherical wall 514 is greater than a radius of the spherical head end 220, i.e., a radius of a sphere in which the spherical wall 514 is located is greater than a radius of a sphere in which the spherical head end 220 is located. A length L of the spherical wall 514 in an axial direction of the first shaft sleeve 510 is less than the depth s of the groove 512.

Specifically, the first shaft sleeve 510 is further provided with a liquid feeding hole 516 in communication with the groove 512. The liquid feeding hole 516 can be in fluid communication with the cleaning pipeline in the catheter 50, so that the flushing liquid can enter the groove 512 through the liquid feeding hole 516. The flushing liquid enters between the groove wall of the groove 512 and the spherical head end 220 to serve as a lubricant, so as to reduce the friction between the spherical head end 220 and the groove wall of the groove 512, thereby reducing the wear of the spherical head end 220 and the groove wall.

Specifically, an opening 516a of the liquid feeding hole 516 is located at a center of the spherical wall 514, so that the flushing fluid entering the groove 512 from the liquid feeding hole 516 can provide an axial impact force to the spherical head end 220 of the rotating shaft 200 as much as possible. More specifically, a central axis of the liquid feeding hole 516 coincides with a central axis of a cavity enclosed by the spherical wall 514, so that the liquid feeding hole 516 is a straight hole to reduce the energy consumption of the flushing liquid in the liquid feeding hole 516.

Specifically, a diameter of the opening 516a of the liquid feeding hole 516 located on the spherical wall 514 is 1/9 to 1/3 of a diameter of a sphere where the spherical head end 220 is located (a diameter of the spherical head end 220 is also the diameter of the sphere where the spherical head end 220 is located). In the illustrated embodiment, since the diameter of the liquid feeding hole 516 is constant, that is, the diameter of the liquid feeding hole 516 is 1/9 to 1/3 of the diameter of the spherical head end 220. If the diameter of the opening 516a of the liquid feeding hole 516 on the spherical wall 514 is too large, a contact surface between the spherical head end 220 and the spherical wall 514 will be reduced, and the wear of the spherical head end 220 by the spherical wall 514 will be increased. If the diameter of the opening 516a is too small, the amount of the flushing liquid entering the groove 512 from the liquid feeding hole 516 will be affected. On the one hand, the flushing liquid entering the groove 512 provides the spherical head end 220 with the impact force, and on the other hand, the flushing liquid enters between the spherical head end 220 and the spherical wall 514 to serve as a lubricant, so as to reduce a friction coefficient between the spherical head end 220 and the spherical wall 514. Therefore, the amount of the flushing liquid entering the groove 512 should not be too small.

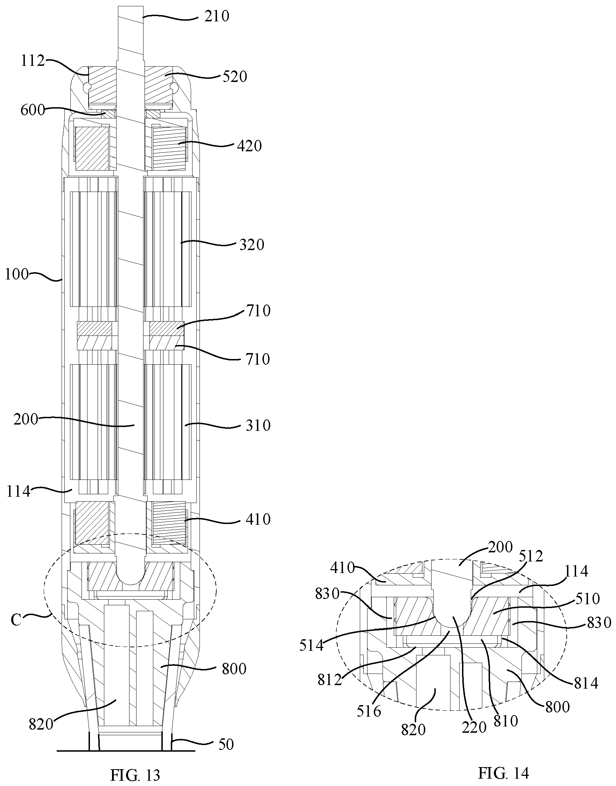

Referring to FIG. 13, FIG. 14 and FIG. 15, specifically, the driving mechanism 10 further includes a supporting base 800 fixedly connected to the housing 100. The supporting base 800 is provided with a mounting cavity 810 and a liquid inlet hole 820 in communication with the mounting cavity 810. The first shaft sleeve 510 is mounted in the mounting cavity 810. The liquid feeding hole 516 is in communication with the liquid inlet hole 820. An end of the liquid inlet hole 820 away from the mounting cavity 810 is configured to be in communication with the cleaning pipeline of the catheter 50, so that the flushing liquid can flow between the groove wall of the groove 512 and the spherical head end 220 through the liquid inlet hole 820 and the liquid feeding hole 516, and then flow into the inner cavity of the housing 100.

Specifically, the mounting cavity 810 includes a cavity bottom 812, and an opening of the liquid inlet hole 812 is located at the cavity bottom 812 of the mounting cavity 810. A supporting step 814 is provided in the mounting cavity 810, and the supporting step 814 abuts against the first shaft sleeve 510, so that the first shaft sleeve 510 is spaced apart from the cavity bottom 812 by a certain distance, so as to better ensure the smooth circulation of the flushing liquid. Specifically, the supporting step 814 abuts against a surface of the first shaft sleeve 510 away from the second shaft sleeve 520.

Specifically, the supporting base 800 is further provided with a branch channel 830, and the branch channel 830 is in fluid communication with the liquid inlet hole 820, so that flushing liquid flowing through the liquid inlet hole 820 can further flow into the inner cavity of the housing 100 through the branch channel 830. Specifically, one end of the branch channel 830 is in communication with a gap between the first shaft sleeve 510 and the cavity bottom 812 of the mounting cavity 810, and the other end of the branch channel 830 is in communication with the accommodating cavity 114. In the illustrated embodiment, the branch channel 830 is formed by partially recessing a cavity wall of the mounting cavity 810. In other words, in a normal state, the flushing liquid is divided into two streams after entering the mounting cavity 810 from the liquid inlet hole 820, one stream flows into the groove 512 of the first shaft sleeve 510 through the liquid feeding hole 516, and the other stream flows out through the branch channel 830. The branch channel 830 can ensure the flow of the flushing liquid when the spherical head end 220 blocks the liquid feeding hole 516. In the illustrated embodiment, two branch channels 830 are provided, and the two branch channels 830 are arranged opposite to each other. It should be understood that the number of the branch channel 830 can be adjusted according to design requirements, for example, in some embodiments, one or more than two branch channels 830 are provided.

Referring to FIG. 2, FIG. 3, FIG. 4 and FIG. 16, the second shaft sleeve 520 abuts against the separating ring 120, i.e., the separating ring 120 is located between the second shaft sleeve 520 and the rotor 400. In the illustrated embodiment, the separating ring 120 is located between the second shaft sleeve 520 and the second rotor 420. The second shaft sleeve 520 can be conveniently positioned by the separating ring 120, so that the assembly of the second shaft sleeve 520 can be facilitated. The second shaft sleeve 520 is provided with a shaft hole 522, and the rotating shaft 200 rotatably extends through the shaft hole 522. In the illustrated embodiment, a central axis of the shaft hole 522 coincides with the central axis of the liquid feeding hole 516 of the first shaft sleeve 510. A gap is provided between A hole wall of the shaft hole 522 of the second shaft sleeve 520 and the rotating shaft 200 for the fluid to flow through. The flushing liquid entering the accommodating cavity 114 can flow out of the housing 100 through the gap between the rotating shaft 200 and the hole wall of the shaft hole 522.

The blocking member 600 is fixedly connected to at least one of the rotating shaft 200 and the rotor 400 (specifically, the second rotor 420). In other words, the blocking member 600 may be directly fixed only to the rotor 400, may be directly fixed only to the rotating shaft 200, or may be directly fixed to both the rotor 400 and the rotating shaft 200. Since the rotor 400 is fixedly connected to the rotating shaft 200, the blocking member 600, the rotating shaft 200, and the rotor 400 rotate and move synchronously. The blocking member 600 is located between the rotor 400 and the second shaft sleeve 520, and the blocking member 600 can abut against the second shaft sleeve 520 to limit the movement of the rotating shaft 200 along the axis of the rotating shaft 200 in a direction approaching the impeller 20.

Since the blocking member 600, the rotating shaft 200, and the rotor 400 rotate and move synchronously. The blocking member 600 can abut against the second shaft sleeve 520 to limit the movement of the rotating shaft 200 along the axis of the rotating shaft 200 in the direction approaching the impeller 20, and the spherical head end 220 of the rotating shaft 200 is provided in the groove 512 of the first shaft sleeve 510 and can abut against the spherical wall 514 of the groove 512, so as to limit a movement range of the rotating shaft 200 along the axis of the rotating shaft 200 in a direction away from the impeller 20, thereby achieving the limitation of the rotating shaft 200 in the axis of the rotating shaft 200. Meanwhile, since the rotating shaft 200 extends through the second shaft sleeve 520, and the spherical head end 220 of the rotating shaft 200 is provided in the groove 512 of the first shaft sleeve 510, the groove wall of the groove 512 of the first shaft sleeve 510 can also limit the swinging range of the spherical head end 220 of the rotating shaft 200 in the radial direction of the rotating shaft 200, thereby achieving overall limitation of the swinging range of the rotating shaft 200 in the radial direction. In other words, the above design not only achieves an axial limit to the rotating shaft 200, but also achieves a radial limit to the rotating shaft 200.

In the illustrated embodiment, the blocking member 600 is fixedly connected to the second rotor 420. Specifically, the blocking member 600 is fixedly connected to the second flywheel 422 of the second rotor 420. In some embodiments, the blocking member 600 is adhered to the second flywheel 422 of the second rotor 420. In some embodiments, the blocking member 600 and the second flywheel 422 of the second rotor 420 are integrally formed. Since the overall volume of the blood pump 1 is small, the volume of the blocking member 600 is smaller, it is difficult to be processed in precision, and has large assembly difficulty, the blocking member 600 and the second flywheel 422 are integrally formed to facilitate the mounting, and the adhering operation is omitted.

Specifically, when the blocking member 600 abuts against the second shaft sleeve 520, the blocking member 600 is at least partially located in an inner ring of the separating ring 120, a gap for fluid to flow is formed between the blocking member 600 and a wall of the inner ring of the separating ring 120, and the separating ring 120 is spaced from the rotor 400 by a certain distance. By providing the gap for fluid to flow between the blocking member 600 and the wall of the inner ring of the separating ring 120, the flushing liquid can flow into the shaft hole 522 of the second shaft sleeve 520 through the gap between the blocking member 600 and the wall of the inner ring of the separating ring 120, that is, the fluid communication between the shaft hole 522 of the second shaft sleeve 520 and the accommodating cavity 114 is achieved. When the blocking member 600 abuts against the second shaft sleeve 520, the separating ring 120 is spaced apart from the rotor 400 by the certain distance, so as to avoid wear due to the friction between the rotor 400 and the separating ring 120 when the blocking member 600 abuts against the second shaft sleeve 520. Specifically, the blocking member 600 is substantially ring-shaped, and a central axis of the blocking member 600 coincides with the axis of the rotating shaft 200. An outer diameter of the blocking member 600 is less than an inner diameter of the separating ring 120, so that the gap for fluid to flow is formed between the blocking member 600 and the wall of the inner ring of the separating ring 120. In other embodiments, the blocking member 600 may also be formed by arranging a plurality of sector rings that are evenly spaced around the rotating shaft 200 for one circumference, or may be understood as being formed by arranging a plurality of sector rings that are circumferentially discretely arranged.

Specifically, a thickness of the blocking member 600 along the axis of the rotating shaft 200 is greater than a thickness of the separating ring 120 along the axis of the rotating shaft 200, so that the separating ring 120 is spaced apart from the rotor 400 by a certain distance when the blocking member 600 abuts against the second shaft sleeve 520. It should be understood that, in some embodiments, the thickness of the blocking member 600 along the axis of the rotating shaft 200 can be less than or equal to the thickness of the separating ring 120 along the axis of the rotating shaft 200. In this case, the rotor 400 (specifically, the second rotor 420) and the blocking member 600 may be spaced apart by a certain distance along the axis of the rotating shaft 200, and the distance is sufficient to ensure that the separating ring 120 and the rotor 400 are spaced apart by a certain distance when the blocking member 600 abuts against the second shaft sleeve 520.

Specifically, a surface of the second shaft sleeve 520 facing the blocking member 600 is partially recessed to form a guiding groove 524, and the guiding groove 524 is in communication with the shaft hole 522 of the second shaft sleeve 520. When the blocking member 600 abuts against the second shaft sleeve 520, part of the guiding groove 524 is not covered by the blocking member 600, so that the blocking member 600 abuts against the second shaft sleeve 520, even if the flow of the flushing liquid is obstructed due to the blocking member 600 blocking the gap between the shaft hole 522 of the second shaft sleeve 520 and the rotating shaft 200, the guiding groove 524 not covered by the blocking member 600 can achieve fluid communication when the blocking member 600 abuts against the second shaft sleeve 520, so as to ensure the smoothness of the flushing liquid. In addition, the guiding groove 524 is formed by partially recessing the surface of the second shaft sleeve 520 facing the blocking member 600, so that the flushing fluid can flow better between the blocking member 600 and the second shaft sleeve 520 to lubricate the contact surface between the blocking member 600 and the second shaft sleeve 520. The friction between the blocking member 600 and the second shaft sleeve 520 is reduced, the wear problem due to the friction between the blocking member 600 and the second shaft sleeve 520 is reduced.

Specifically, the blocking member 600 has a blocking surface 610 perpendicular to the axis of the rotating shaft 200. The second shaft sleeve 520 has a stopping surface 526 perpendicular to the central axis of the shaft hole 522 of the second shaft sleeve 520. The stopping surface 526 is opposite to the blocking surface 610, and the stopping surface 526 can abut against the blocking surface 610, so as to limit the movement of the rotating shaft 200 along the axis of the rotating shaft 200 in the direction approaching the impeller 20. Since the blocking surface 610 is perpendicular to the axis of the rotating shaft 200, and the stopping surface 526 is perpendicular to the central axis of the shaft hole 522 of the second shaft sleeve 520, the rotating shaft 200 rotatably extends through the shaft hole 522 of the second shaft sleeve 520, so that when the rotating shaft 200 operates normally and the blocking member 600 abuts against the second shaft sleeve 520, the blocking surface 610 and the stopping surface 526 can be in contact with each other, which can reduce the wear caused by the friction between the blocking member 600 and the second shaft sleeve 520. Specifically, the stopping surface 526 abuts against the separating ring 120. The guiding groove 524 is formed by partially recessing the stopping surface 526.

Specifically, a roughness of at least one of the blocking surface 610 and the stopping surface 526 is less than or equal to 0.1 microns. In some embodiments, the roughness of both the blocking surface 610 and the stopping surface 526 is less than or equal to 0.1 microns. In some embodiments, the roughness of one of the blocking surface 610 and the stopping surface 526 is less than or equal to 0.1 microns. By reducing the roughness of at least one of the blocking surface 610 and the stopping surface 526, the friction between the blocking surface 610 and the stopping surface 526 can be effectively reduced, and the wear problem due to the friction between the second shaft sleeve 520 and the blocking member 600 can be reduced.

In some embodiments, at least one of the blocking surface 610 and the stopping surface 526 is a ceramic surface. Ceramic has high machining accuracy, high biocompatibility, high mechanical strength, good wear resistance and corrosion resistance. In this case, the blocking member 600 and the second shaft sleeve 520 may be made of ceramic, or at least one of the blocking surface 610 and the stopping surface 526 may be a ceramic surface by providing a ceramic coating. In some embodiments, the blocking surface 610 is made of diamond, so that the blocking surface 610 has a relatively high hardness, a relatively smooth surface, and is wear-resistant. In this case, the material of the blocking surface 610 is a ceramic surface by providing a diamond coating.

It should be understood that the structure of the driving mechanism 10 is not limited to the above structure. In some embodiments, one rotor 400 and one stator 300 are provided. In this case, the rotor 400 is adjacent to the second shaft sleeve 520, and the stator unit is adjacent to the first shaft sleeve 510. In some embodiments, the rotor 400 still has the first rotor 410 and the second rotor 420, but the stator 300 has one stator unit. In this case, the stator unit is located between the first rotor 410 and the second rotor 420, and the stator unit is capable of simultaneously driving the first rotor and the second rotor to rotate.

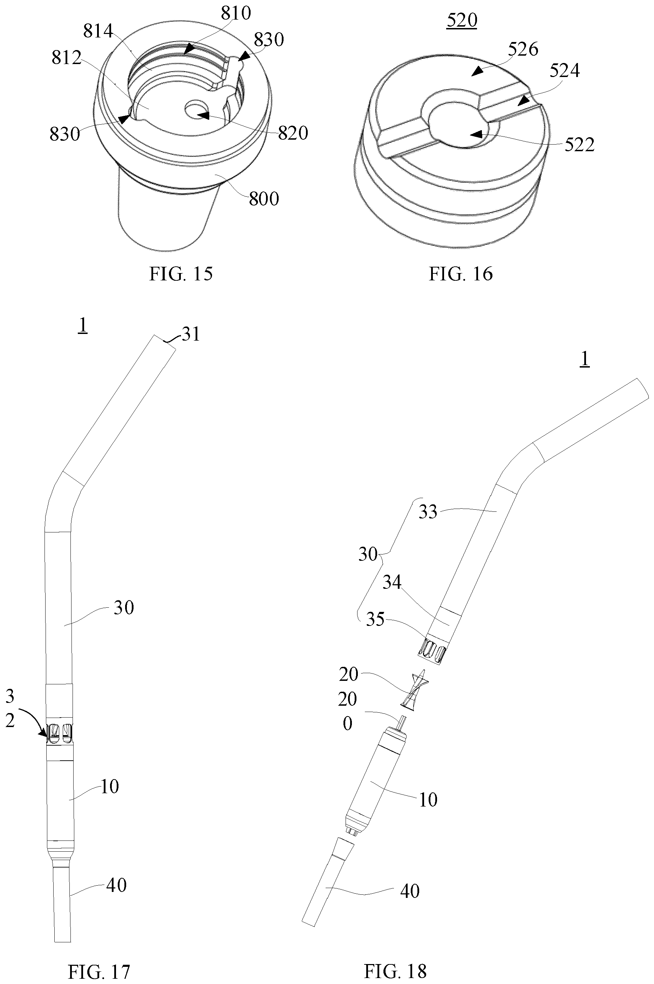

Referring to FIGS. 17 and 18, the blood pump 1 includes a driving mechanism 10 and an impeller 20. The driving mechanism 10 is in transmission connection with the impeller 20, and the driving mechanism 10 is capable of driving the impeller 20 to rotate. Specifically, the blood pump 1 further includes a cannula assembly 30 fixedly connected to a distal end of the driving mechanism 10. The impeller 20 is rotatably accommodated in the cannula assembly 30. The cannula assembly 30 has a blood inlet 31 and a blood outlet 32. The blood inlet 31 is provided at a distal end of the cannula assembly 30, and the blood outlet 32 is provided at a proximal end of the cannula assembly 30. A plurality of blood outlets 32 are provided, and the plurality of the blood outlets 32 are spaced circumferentially along the cannula assembly 30. When the impeller 20 rotates, blood flows from the blood inlet 31 into the cannula assembly 30 and then flows out of blood outlet 32. In an embodiment, the cannula assembly 30 extends through a heart valve, such as an aortic valve. The blood inlet 31 is located within a heart, and the blood outlet 32 and the driving mechanism 10 are located in a blood vessel, such as an aorta outside a heart.

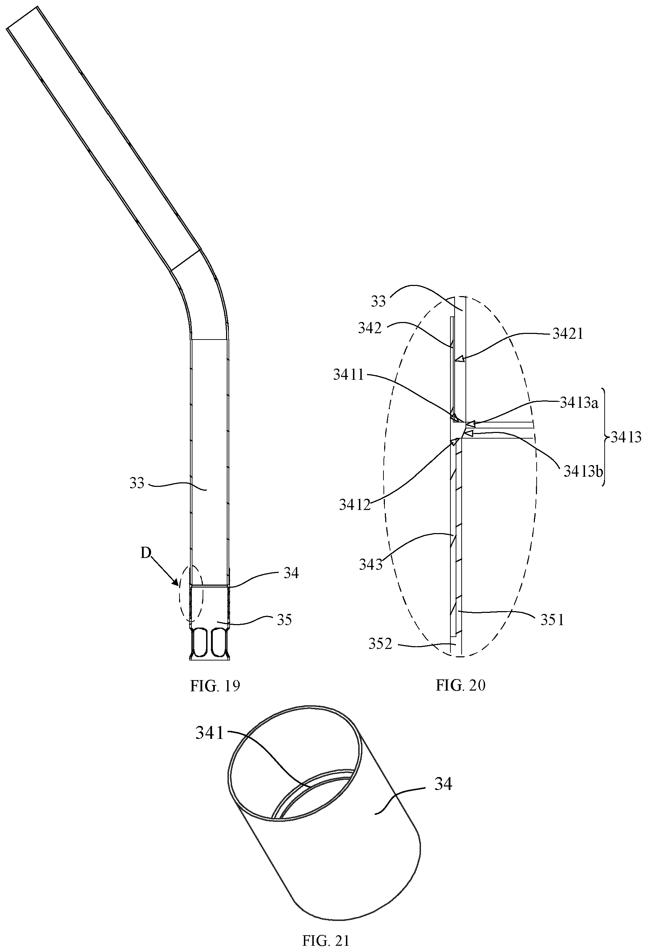

Referring to FIG. 19, the cannula assembly 30 includes an inserting tube 33, a connecting tube 34, and an outlet tube 35. The inserting tube 33, the connecting tube 34, and the outlet tube 35 are hollow tubular structures. The inserting tube 33 is sleeved in the connecting tube 34, so that an inner wall of one end of the connecting tube 34 is connected to an outer wall of the inserting tube 33. Specifically, the inserting tube 33 has a proximal end and a distal end. The proximal end of the inserting tube 33 is engaged with the connecting tube 34. The blood inlet 31 is located on the distal end of the inserting tube 33.

Referring to FIG. 20, the outlet tube 35 includes a connecting portion 351 adjacent to the connecting tube 34 and an outlet portion 352 away from the connecting tube 34. The outlet tube 35 is sleeved in the connecting tube 34, so that an outer wall of the connecting portion 351 is connected to an inner wall of an end of the connecting tube 34 away from the inserting tube 33. An end of the outlet portion 352 away from the connecting tube 34 is fixedly connected to the driving mechanism 10, and the impeller 20 is rotatably provided in the outlet tube 35, or the impeller 20 is partially provided in the outlet tube 35 and partially provided in the inserting tube 33. The blood outlet 32 is located on the outlet portion 352. The plurality of blood outlets 32 are uniformly arranged circumferentially on the outlet portion 352 of the outlet tube 35, and the impeller 20 is driven to rotate by the driving mechanism 10 to allow blood to flow into the blood inlet 31, flow through the inserting tube 33, and then flow out from the plurality blood outlets 32 of the outlet tube 35.

Since the conventional connection mode between the inserting tube 33 and the outlet tube 35 is that the inserting tube 33 is directly sleeved on the outlet tube 35, but the inserting tube 33 is a flexible tube and the outlet tube 35 is a metal tube, there is no matching assembly position, and diameters of the inserting tube 33 and the outlet tube 35 are different. Therefore, the inserting tube 33 before connection needs to be shaped by a process to expand a diameter of an opening of the inserting tube 33 to fit the outlet tube 35, so that the inserting tube 33 can be sleeved on the outer wall of the outlet tube 35. The process is complicated, the connection is unstable, and the strength of a shaping portion is reduced.

The inserting tube 33 and the outlet tube 35 of the cannula assembly 30 provided in this embodiment are smoothly connected through the connecting tube 34. The inner wall of one end of the connecting tube 34 is connected to the outer wall of the inserting tube 33, and the outer wall of the connecting portion 351 of the outlet tube 35 is connected to the inner wall of the other end of the connecting tube 34, so that the inserting tube 33 and the outlet tube 35 can be fixedly connected to form a continuous pipeline. A step of shaping the insert tube 33 by a process due to directly sleeve-connecting the insert tube 33 and the outlet tube 35 is omitted, the connection is stable and the assembly operation is simple.

As shown in FIGS. 19 to 22, the inner wall of the connecting tube 34 is provided with a limiting protruding ring 341. Specifically, the limiting protruding ring 341 is in an annular shape protruding from the inner wall of the connecting tube 34, and the limiting protruding ring 341 is coaxial with the connecting tube 34. The limiting protruding ring 341 has a first end surface 3411 and a second end surface 3412 that are arranged along an axial direction of the connecting tube 34. The first end surface 3411 abuts against an end portion of the inserting tube 33, and the second end surface 3412 abuts against an end portion of the connecting portion 351. An outer diameter of the connecting portion 351 is less than an outer diameter of the outlet portion 352. An end portion of the connecting tube 34 away from the inserting tube 33 abuts against an end portion of the outlet portion 352 adjacent to the connecting portion 351, and an outer wall of the connecting tube 34 is coplanar with an outer wall of the outlet portion 352. Specifically, inner diameters of the connecting portion 351 and the outlet portion 352 are the same. The limiting protruding ring 341 has a certain thickness to form the first end surface 3411 adjacent to the inserting tube 33 and the second end surface 3412 adjacent to the outlet tube 35. When the inserting tube 33 and the outlet tube 35 are respectively accommodated in the connecting tube 34, the limiting protruding ring 341 can simultaneously limit the axial direction of the outlet tube 35 and the inserting tube 33, so as to control a depth of the sleeve connection between the outlet tube 35 and the inserting tube 33, serves to pre-position, and facilitate the subsequent fixing operation. At the same time, the end of the connecting tube 34 can abut against the end of the outlet portion 352, and cooperate with the limiting protruding ring 341 to form limiting in two directions in the axial direction, which is more stable and fit. At this time, the outer wall of the connecting tube 34 is coplanar with the outer wall of the outlet portion 352, so that the outer wall of the connecting tube 34 and the outer wall of the outlet portion 352 of the outlet tube 35 are on the same cylindrical surface, and the overall outer diameter of the connection between the connecting tube 34 and the outlet tube 35 is effectively controlled, so that the connection is smooth, and the presence of an undulating drop section that causes difficulty in entering the human blood vessels or even scratching the blood vessels is avoided.

In addition, as shown in FIGS. 18 to 20 and FIG. 24, the impeller 20 includes a blade 21. An end portion of the blade 21 away from the driving mechanism 10 does not extend beyond the end portion of the connecting portion 351 away from the driving mechanism 10. Specifically, the impeller 20 further includes a hub 22, and the blades 21 are spirally wound around the hub 22. The impeller 20 is partially positioned in the outlet tube 35, and the driving mechanism 10 drives the impeller 20 to rotate. Since the impeller 20 will vibrate and generate deflection when rotating, a certain distance needs to be provided between the impeller 20 and an inner wall of the outlet tube 35 to ensure that the blade 21 will not collide with the inner wall of the outlet tube 35 when the impeller 20 generates the maximum deflection. An inner wall of the tube at a position where the limiting protruding ring 341 is located is narrow, while the blade 21 has a certain height in an axial direction of the outlet tube 35, the blade 21 occupies a certain width in a radial direction of the outlet tube 35, and the highest point of the blade 21 in the axial direction does not protrude from the outlet tube 35. In this way, the blade 21 will not in contact with the limiting protruding ring 341. Moreover, the limiting protruding ring 341 is not limited to the complete circular ring structure in the above embodiment, and can also be a plurality of boss structures that are not connected to each other and are uniformly spaced, which can save materials while ensuring the limiting. The limiting protruding ring 341 may be a structure separately connected to the connecting tube 34 or may be a structure integrally formed with the connecting tube 34, and the strength is higher if the limiting protruding ring 341 is integrally formed with the connecting tube 34.

As shown in FIG. 20, an inner diameter of the inserting tube 33 is less than an inner diameter of the outlet tube 35, and a width of the first end surface 3411 in the radial direction is greater than a width of the second end surface 3412 in the radial direction, so that an inner wall of the inserting tube 33 is coplanar with an edge of the first end surface 3411, and an inner wall of the outlet tube 35 is coplanar with an edge of the second end surface 3412. The limiting protruding ring 341 further includes a transition surface 3413 connected to the edge of the first end surface 3411 and the edge of the second end surface 3412. Specifically, the inserting tube 33 and the outlet tube 35 are of different sizes and specifications. Since the inner diameters of the two are different, i.e., the first end surface 3411 protrudes more from the inner wall of the connecting tube 34 in the radial direction than the second end surface 3412. When the first end surface 3411 and the second end surface 3412 abut against the limiting protruding ring 341 respectively, the edge of the first end surface 3411 needs to be coplanar with the inner wall of the inserting tube 33, and the edge of the second end surface 3412 needs to be coplanar with the inner wall of the outlet tube 35. In this way, after the outlet tube 35 and the inserting tube 33 abut against the limiting protruding ring 341, a blood flow channel formed by the inner walls of the outlet tube 35, the inserting tube 33 and the limiting protruding ring 341 is smoother, and no dead corner will be formed at the connection position between of the outlet tube 35, the inserting tube 33 and the limiting protruding ring 341, so that blood is prevented from entering the dead corner to cause blood blockage and form thrombus.

More specifically, since the widths of the first end surface 3411 and the second end surface 3412 are different, a distance between the first end surface 3411 and the second end surface 3412 will form a large vertical drop, and a direction of blood flow is from the inserting tube 33 to the outlet tube 35, and a dead corner will be formed between the two, so that the edge of the first end surface 3411 and the edge of the second end surface 3412 are connected through the transition surface 3413 to form a smooth transition surface 3413, so that the blood can flow directly along the transition surface 3413 without forming a dead corner to cause the risk of blood jamming and thrombus.

The transition surface 3413 may be provided as an outer convex curved surface or an inner concave curved surface, and the curved surface has a better effect on guiding blood.

As shown in FIG. 20, the transition surface 3413 has a cylindrical surface 3413a that is coaxial with the connecting tube 34 and an inclined surface 3413b that is at an angle to the axis of the connecting tube 34. An end of the cylindrical surface 3413a is connected to an end of the inclined surface 3413b, and an end of the cylindrical surface 3413a away from the inclined surface 3413b is connected to the edge of the first end surface 3411. The cylindrical surface 3413a is coplanar with the inner wall of the inserting tube 33, and an end of the inclined surface 3413b away from the cylindrical surface 3413a is connected to the edge of the second end surface 3412.

The transition surface 3413 is composed of two parts. One end of the cylindrical surface 3413a is connected to the edge of the first end surface 3411, and the inserting tube 33 is coplanar with the inner wall of the inserting tube 33 after abutting against the first end surface 3411. Coplanar refers to a smooth transition of the connection surface between the cylindrical surface 3413a and the inserting tube 33 without undulating drop. When the limiting protruding ring 341 is processed and formed, the transition is made through the cylindrical surface 3413a, so as to prevent the inclined surface 3413b from being directly connected to the edge of the first end surface 3411 to form a sharp corner structure at the connection, so that the processing difficulty is low. Then, the cylindrical surface 3413a and the edge of the second end surface 3412 are connected through the inclined surface 3413b. At this time, the blood flow flows through the cylindrical surface 3413a and is guided along the inclined surface 3413b to flow to the outlet tube 35. Therefore, the inserting tube 33 and the outlet tube 35 with different inner diameters are connected through the limiting protruding ring 341, so that the blood flow channel forms a continuous channel. The blood flow flows from the inner wall of the inserting tube 33 to the transition surface 3413 of the limiting protruding ring 341, and then flows into the inner wall of the outlet tube 35. The whole process is unobstructed to avoid blood jamming and thrombosis.

Further, the outlet tube 35 is a metal tube. The inserting tube 33 is a flexible tube and has elasticity. In this embodiment, the outlet tube 35 is specifically a plastic tube, and the connecting tube 34 is also a metal tube. Since two ends of the connecting tube 34 need to be bonded with two different materials respectively, in the bonding process, an inner wall of an end of the connecting tube 34 is adhered to the outer wall of the inserting tube 33, and an end of the connecting tube 34 away from the inserting tube 33 is welded to the outer wall of the connecting portion 351, which meets the process requirements. Moreover, the connecting tube 34 is sleeved at the joint between the inserting tube 33 and the outlet tube 35, so that the strength is also ensured.

As shown in FIG. 20, the connecting tube 34 includes a first tube portion 342 and a second tube portion 343 connected to each other. An inner wall of the first tube portion 342 is connected to the outer wall of the inserting tube 33, and an inner wall of the second tube portion 343 is connected to the outer wall of the connecting portion 351. The limiting protruding ring 341 is provided on the inner wall of the second tube portion 343. Outer diameters of the first tube portion 342 and the second tube portion 343 are the same, and an inner diameter of the first tube portion 342 is greater than an inner diameter of the second tube portion 343. The connecting tube 34 is divided into two sections based on a thickness of a tube wall thereof. The first tube portion 342 having a relatively thin thickness is connected to the inserting tube 33, and the second tube portion 343 having a relatively thick thickness is connected to the outlet tube 35. Since the inserting tube 33 is a flexible tube and has elasticity, it is easier to enter the curved blood vessel of the human body by relying on such characteristics, while the connecting tube 34 is a metal tube with a hard material, and the connecting tube 34 is sleeved on the outer wall of the inserting tube 33 in the axial direction thereof, which makes the nesting portion too rigid. The elasticity of the first tube portion 342 is improved by reducing the thickness of the first tube portion 342, so that the first tube portion 342 is not be too hard and can adapt to deformation when entering the curved blood vessels of the human body.

Further, a tube portion gap 3421 is provided between the inner wall of the first tube portion 342 and the outer wall of the inserting tube 33. The certain tube portion gap 3421 is reserved in the radial direction for glue dispensing operation between the first tube portion 342 and the inserting tube 33. At the same time, outer wall of the first tube portion 342 will not expand outward after the predetermined glue is filled, so that an outer diameter of the glue dispensing position is prevented from expanding.

Due to the existence of the tube portion gap 3421, after the inserting tube 33 and the connecting tube 34 are sleeved, the concentricity of the inserting tube 33 and the connecting tube 34 cannot be ensured, so that in the specific operation, an auxiliary positioning tool needs to be used to realize the concentricity of the inserting tube 33 and the connecting tube 34. The auxiliary positioning tool may be a cylindrical positioning column, and an outer diameter of the positioning column is the same as the inner diameter of the cylindrical surface 3413a of the inserting tube 33 or the limiting protruding ring 341. Specifically, the positioning column is first inserted to enable an outer wall of the positioning column to be attached to the cylindrical surface 3413a of the limiting protruding ring 341, then the inserting tube 33 is inserted to enable the inner wall of the inserting tube 33 to be attached to the outer wall of the positioning column, and then glue is dispensed in the tube portion gap 3421 between the inserting tube 33 and the connecting tube 34.

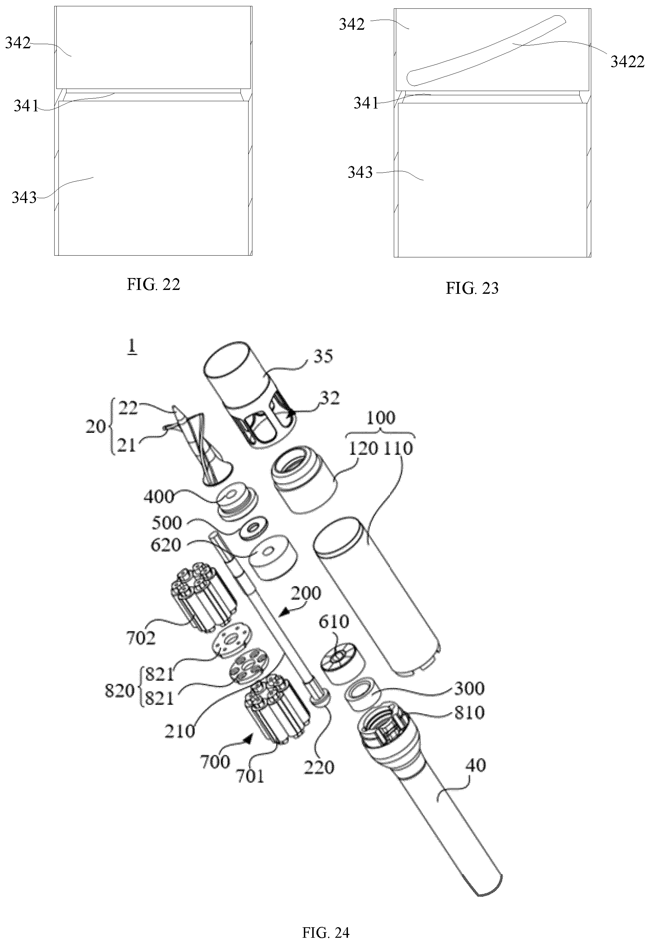

Further, as shown in FIG. 23, the first tube portion 342 is provided with a plurality of hollow grooves 3422. The hollow grooves 3422 are through grooves extending through the inner and outer walls of the first tube portion 342, which can effectively reduce an area of the first tube portion 342, thereby improving the elasticity of the first tube portion 342. Meanwhile, during the glue dispensing process, glue may be dispensed in the hollow groove 3422 on a side surface of the first tube portion 342. The glue dispensing can be operated in multiple directions to make the glue dispensing more uniform and achieve better bonding effect. In addition, when the glue is heated and dried, the glue will expand. If the side surface of the first tube portion 342 is a closed space, the outer diameter will be extended when the glue expands. At this time, the hollow groove 3422 can also accommodate a part of the glue, so that the glue has an expandable space and does not cause the outer diameter to expand. The hollow groove 3422 may be an I-shaped straight groove, or an S-shaped or J-shaped curved groove. In the arrangement, the hollow grooves 3422 may be arrayed along the radial direction of the connecting tube 34 or along the axial direction of the connecting tube 34.

Specifically, referring to FIGS. 17 and 24, the blood pump 1 further includes a catheter 40 connected to the proximal end of the driving mechanism 10. The catheter 40 is configured to accommodate various supply lines. For example, the supply lines include a wire configured to be electrically connected to the driving mechanism 10 and a cleaning pipeline configured to supply flushing fluid to the driving mechanism 10 of the blood pump 1. Optionally, the flushing fluid is physiological saline, physiological saline containing heparin, or glucose, etc.

Referring to FIGS. 24 to 26, the driving mechanism 10 includes a housing 100, a rotating shaft 200, a first shaft sleeve 300, a second shaft sleeve 400, and a blocking member 500. A distal end of the housing 100 is fixedly connected to the cannula assembly 30, and a proximal end of the housing 100 is fixedly connected to the catheter 40. The housing 100 is substantially a cylindrical housing with openings at both ends thereof. The housing 100 has an inner cavity 101. The flushing liquid in the cleaning line flows into the inner cavity 101 through the proximal end of the housing 100 and flows out of the housing 100 from the distal end of the housing 100.

In some embodiments, the housing 100 includes a first housing 110 and a second housing 120. A proximal end of the first housing 110 is fixedly connected to the catheter 40, a distal end of the first housing 110 is fixedly connected to a proximal end of the second housing 120, and a distal end of the second housing 120 is fixedly connected to the cannula assembly 30. The first housing 110 and the second housing 120 enclose to form the interior cavity 101 of the housing 100. The housing 100 is formed by splicing the first housing 110 and the second housing 120, so that the rotating shaft 200, the first shaft sleeve 300, the second shaft sleeve 400, the blocking member 500, etc., are conveniently mounted in the inner cavity 101 of the housing 100.

The rotating shaft 200 is rotatably provided in the housing 100. The rotating shaft 200 is fixedly connected to the impeller 20 to drive the impeller 20 to rotate. The rotating shaft 200 includes a shaft portion 210 and a sliding portion 220. The shaft portion 210 is rotatably mounted to the housing 100, one end of the shaft portion 210 is fixedly connected to the sliding portion 220, and the other end of the shaft portion 210 is configured to be connected to the impeller 20. The shaft portion 210 is elongated. A distal end of the shaft portion 210 is located outside the housing 100 and configured to be fixedly connected to the impeller 20. The impeller 20 can rotate along with the shaft portion 210. In the illustrated embodiment, the shaft portion 210 extends substantially along an axial direction of the housing 100, in other words, an axis of the shaft portion 210 extends in a direction substantially consistent with the axial direction of the housing 100.

The sliding portion 220 has a spherical crown surface 221. The axis of the shaft portion 210 extends through a center of a sphere where the spherical crown surface 221 is located. In the illustrated embodiment, a diameter of the sphere where the spherical crown surface 221 is located is greater than a diameter of the shaft portion 210. Specifically, a height of the spherical crown surface 221 in the axial direction of the shaft portion 210 is greater than or equal to a radius of the sphere where the spherical crown surface 221 is located. In other words, a surface area of the spherical crown surface 221 is at least half of a surface area of the sphere where the spherical crown surface 221 is located.

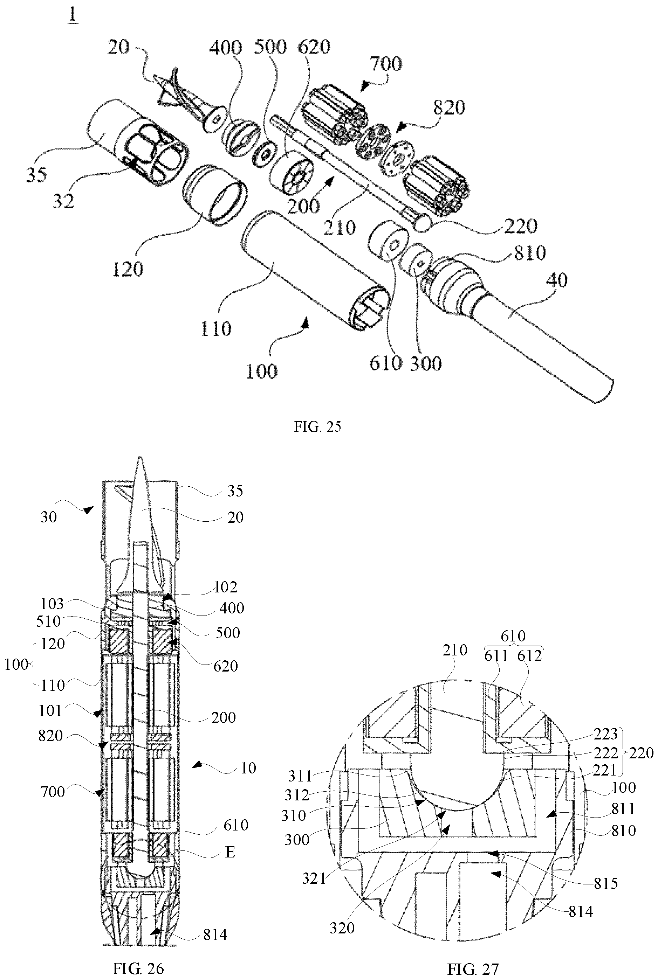

Referring to FIG. 27, in the illustrated embodiment, the sliding portion 220 further includes a cylindrical surface 222 and a limiting surface 223. One end of the cylindrical surface 222 is connected to the spherical crown surface 221, and the other end of the cylindrical surface 222 is connected to the limiting surface 223. An axis of the cylindrical surface 222 coincides with the axis of the shaft portion 210. The limiting surface 223 is perpendicular to the axis of the shaft portion 210. The limiting surface 223 is substantially circular, a central axis of the cylindrical surface 222 passes through the center of the sphere where the spherical crown surface 221 is located, and passes through a center of the limiting surface 223. In the illustrated embodiment, a diameter of the cylindrical surface 222 is equal to the diameter of the sphere where the spherical crown surface 221 is located.

It should be understood that the sliding portion 220 is not limited to the above structure. In some embodiments, the sliding portion 220 may have a spherical structure as a whole. In this case, the spherical crown surface 221 is a partial surface of the sliding portion 220 away from the shaft portion 210. In this case, the sliding portion 220 does not have the limiting surface 223 and the cylindrical surface 222. Alternatively, the sliding portion 220 does not have the cylindrical surface 222, and the limiting surface 223 is directly connected to the spherical crown surface 221. In some embodiments, the shaft portion 210 and the sliding portion 220 are integrally formed. In some embodiments, the shaft portion 210 and the sliding portion 220 may be fixedly connected together by assembling, welding, adhering, etc. In some embodiments, the sliding portion 220 includes a sliding body and a diamond coating provided on a surface of the sliding body, so that the surface of the sliding portion 220 is smooth and has high wear resistance. In this case, the material of the sliding body may be a material having a certain rigidity, such as metal, ceramic, etc., and the material of the sliding body may be the same as that of the shaft portion 210.

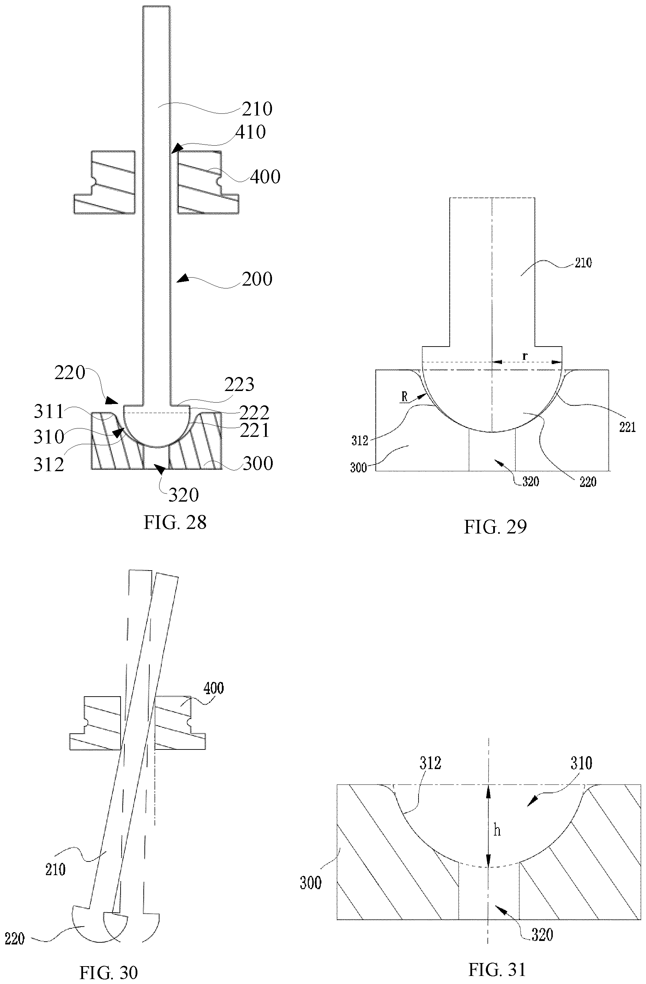

Referring to FIGS. 26 and 28, in the illustrated embodiment, the first shaft sleeve 300 and the second shaft sleeve 400 are both located in the inner cavity 101 of the housing 100 and are spaced apart along the axial direction of the housing 100; The first shaft sleeve 300 is located at the proximal end of the housing 100, and the second shaft sleeve 400 is located at the distal end of the housing 100. The first shaft sleeve 300 is provided with a groove 310, and the sliding portion 220 of the rotating shaft 200 is movably provided in the groove 310, so that the first shaft sleeve 300 limits the movement of the rotating shaft 200 in a direction approaching the first shaft sleeve 300. The shaft portion 210 of the rotating shaft 200 rotatably extends through the second shaft sleeve 400, and the second shaft sleeve 400 limits the movement range of the shaft portion 210 in a radial direction of the shaft portion 210. The second shaft sleeve 400 is provided with a shaft hole 410, and the shaft portion 210 of the rotating shaft 200 rotatably extends through the shaft hole 410. A diameter of the shaft hole 410 is slightly greater than a diameter of a portion of the shaft portion 210 of the rotating shaft 200 located in the shaft hole 410, so as to allow the shaft portion 210 to rotate and to allow the flushing liquid to flow through. The shaft hole 410 has a certain length along a central axis of the shaft hole 410 (in other word, the shaft hole 410 has a certain length in the axial direction of the housing 100) to limit a radial swinging range of the shaft portion 210 while limiting a radial swinging range of the sliding portion 220. By adjusting a size of the diameter of the shaft hole 410 and adjusting the length of the shaft hole 410 along the central axis thereof, the radial swinging range of the sliding portion 220 can be adjusted.

When the blood pump 1 operates, the rotating shaft 200 will generate a certain amplitude of swing, as shown in FIG. 30, especially the end of the rotating shaft 200 away from the impeller 20 or the end of the rotating shaft 200 fitted with the first shaft sleeve 300 has a relatively large swing amplitude, and the conventional rotating shaft 200 has the risk of being stuck by the first shaft sleeve 300, while in this embodiment, referring to FIGS. 29 and 31, the spherical crown surface 221 slidably abuts against a groove wall of the groove 310, that is, the sliding portion 220 swings in the groove 310 through the spherical crown surface 221, and a depth h of the groove 310 is less than or equal to the height of the spherical crown surface 221 in the axial direction of the shaft portion 210, so as to effectively prevent the rotating shaft 200 from being stuck due to the contact between the other parts of the rotating shaft 200 except the spherical crown surface 221 and a notch of the groove 310, thereby improving the safety and reliability of the driving mechanism 10 and the blood pump 1. If the depth h of the groove 310 is greater than the height of the spherical crown surface 221 in the axial direction of the shaft portion 210, a portion of the shaft portion 210 is accommodated in the groove 310, and when the rotating shaft 200 swings radially, there is a risk that the rotating shaft 200 will be stuck by the groove 310 and cannot rotate to cause the pump to stop.

Specifically, the groove 310 is a ball groove. The groove 310 has a spherical wall 312, and the spherical crown surface 221 of the sliding portion 220 slidably abuts against the spherical wall 312. A radius R of a sphere where the groove 310 is located (in other words, a sphere where the spherical wall 312 is located) is greater than the radius r of the sphere where the spherical crown surface 221 is located, so that the sliding portion 220 can slide in the groove 310 and has a certain radial swing space. A difference between the radius R of the sphere where the spherical wall 312 is located and the radius r of the sphere where the spherical crown surface 221 is located is defined as D, and 0.04 mm ≤ D ≤ 0.06 mm. By limiting the range of D, on the one hand, the process difficulty of assembling the sliding portion 210 into the groove 310 can be reduced, and on the other hand, the maximum swing angle of the rotating shaft 200 can be limited, thereby ensuring the smooth operation of the driving mechanism 10.

Specifically, the diameter of the groove 310 gradually increases along the axis of the first shaft sleeve 300 and in a direction approaching the second shaft sleeve 400. In other words, the entire groove wall of the groove 310 is almost the spherical wall 312, so that the spherical crown surface 221 of the sliding portion 220 can slide more smoothly in the groove 310. A center of the sphere where the spherical wall 312 is located is located on a central axis of the groove 310. In the illustrated embodiment, the central axis of the groove 310 coincides with a central axis of the first shaft sleeve 300. The central axis of the groove 310 coincides with the central axis of the shaft hole 410. It should be noted that, in the present application, the depth h of the groove 310 refers to the maximum distance from the groove wall of the groove 310 to a plane where the notch of the groove 310 is located when the spherical wall 312 of the groove 310 is in a complete state (i.e., when the spherical wall 312 is in a state of being unperforated) as shown in FIG. 31. That is, the depth h of the groove 310 is a height of a missing portion of the sphere where the spherical wall 312 is located (a bottom surface of the missing portion of the sphere coincides with the plane where the notch of the groove 310 is located).

In an embodiment, the depth h of the groove 310 is 0.6 to 1 times the radius R of the sphere where the spherical wall 312 is located, i.e., 0.6R ≤ h ≤ R. The range not only enables the groove 310 to have a radial limiting effect to prevent the sliding portion 220 from sliding out of the groove 310, but also enables the diameter of the groove 310 to gradually increase along the axis of the first shaft sleeve 300 and in the direction approaching the second shaft sleeve 400, so as to facilitate the sliding portion 220 to be mounted in the groove 310, and such that the groove 310 has an appropriate width to meet the requirement of the radial swinging range of the sliding portion 220.

In an embodiment, referring to FIG. 28 to FIG. 31, the depth h of the groove 310 is greater than or equal to a half of the height of the spherical crown surface 221 in the axial direction of the shaft portion 210, so that the groove wall of the groove 310 has a sufficient radial width for the sliding portion 220 to slide to prevent the sliding portion 220 from sliding out of the groove 310.

In an embodiment, an edge of the notch of the groove 310 is rounded, so that the edge of the notch of the groove 310 forms a first rounded corner 311 to prevent the sliding portion 220 from being scratched and worn by the edge of the notch having sharp corners. In the embodiment shown in FIG. 28, the first rounded corner 311 is located at a distal end of the spherical wall 312, so that the groove wall of the groove 310 is smoothly connected from the spherical wall 312 to an end surface of a distal end of the first shaft sleeve 300.

In some embodiments, referring to FIG. 26 and FIG. 27, the first shaft sleeve 300 is further provided with a flushing liquid hole 320 for flushing liquid to flow therethrough. The flushing liquid hole 320 is in fluid communication with the groove 310. A diameter of the flushing liquid hole 320 is less than the diameter of the sphere where the spherical wall 312 is located. Specifically, an opening at one end of the flushing liquid hole 320 is located on an end surface of a proximal end of the first shaft sleeve 300, and an opening at the other end of the flushing liquid hole 320 is located on the spherical wall 312. The flushing liquid hole 320 can be in communication with a flushing line in the catheter 40 and thus can be in fluid communication with the flushing line, so that the flushing fluid can enter the groove 310 through the flushing liquid hole 320. The diameter of the flushing liquid hole 320 is less than the diameter of the sphere where the spherical crown surface 221 is located.

Specifically, a central axis of the flushing liquid hole 320 passes through the center of the groove 310 or the center of the sphere where the spherical wall 312 is located, so that the flushing liquid can well enter between the groove wall of the groove 310 and the sliding portion 220, which not only serve as a lubricant to reduce the friction coefficient between the sliding portion 220 and the groove wall of the groove 310, in other words, to reduce the friction coefficient between the spherical crown surface 221 and the spherical wall 312 is reduced, thereby reducing the wear of the sliding portion 220 and the first shaft sleeve 300. In addition, the flushing liquid entering the groove 310 from the flushing liquid hole 320 can also exert a hydraulic suspension supporting effect on the sliding portion 220. The flushing fluid then flows out of the groove 310 through the opening of the groove 310 and enters the inner cavity 101 of the housing 100. The flushing liquid hole 320 is a straight hole, so as to reduce the energy consumption of the flushing liquid in the flushing liquid hole 320.

In an embodiment, the flushing liquid hole 320 is provided with a first opening 321 located at an end of the flushing liquid hole 320 adjacent to the groove 310. A diameter of the first opening 321 is 1/9 to 1/3 of a diameter of the spherical crown surface 221. If the diameter of the first opening 321 of the flushing liquid hole 320 is too large, a contact surface between the sliding portion 220 and a side wall of the groove 310 will be reduced (resulting in a larger pressure per unit area), and the wear of the sliding portion 220 by the groove wall of the groove 310 will be increased. If the diameter of the first opening 321 is too small, the amount of the flushing liquid entering the groove 310 from the flushing liquid hole 320 will be affected. On the one hand, the flushing liquid entering the flushing liquid hole 320 provides the sliding portion 220 with the impact force, and at the same time, the flushing liquid enters between the sliding portion 220 and the groove wall of the groove 310 to serve as a lubricant, so as to reduce a friction coefficient between the sliding portion 220 and the groove wall of the groove 310. Therefore, the amount of the flushing liquid entering the groove 310 should not be too small. In addition, an edge of the first opening 321 of the flushing liquid hole 320 is provided with a second rounded corner to prevent the sliding portion 220 from being scratched and worn. In the illustrated embodiment, the first opening 321 is located on the spherical wall 312. The flushing fluid enters between the spherical wall 312 and the spherical crown surface 221 through the first opening 321 for lubrication.

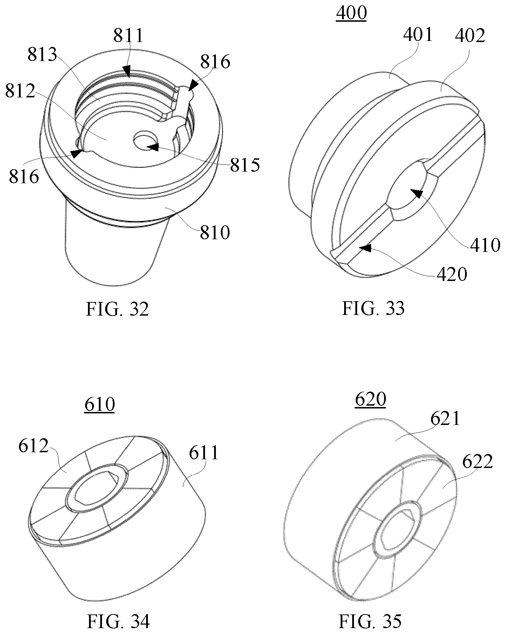

In some embodiments, referring to FIGS. 27 and 32, the driving device 10 further includes a supporting base 810 fixedly connected to the housing 100. The supporting base 810 is provided with a mounting cavity 811 and a liquid inlet hole 814 in communication with the mounting cavity 811. The first shaft sleeve 300 is mounted in the mounting cavity 811. The flushing liquid hole 320 is in communication with the liquid inlet hole 814. An end of the liquid inlet hole 814 away from the mounting cavity 811 is configured to be in communication with the cleaning pipeline of the catheter 40, so that the flushing liquid can flow between the groove wall of the groove 310 and the sliding portion 220 through the liquid inlet hole 814 and the flushing liquid hole 320, and then flow into the inner cavity 101 of the housing 100.

In an embodiment, the mounting cavity 811 has a cavity bottom 812, and a second opening 815 of the liquid inlet hole 814 is located at the cavity bottom 812 of the mounting cavity 811. A supporting step 813 is provided in the mounting cavity 811, and the supporting step 813 abuts against the first shaft sleeve 300, so that the first shaft sleeve 300 is spaced apart from the cavity bottom 812 by a certain distance, so as to better ensure the smooth circulation of the flushing liquid. Specifically, the supporting step 813 abuts against a surface of the first shaft sleeve 300 away from the second shaft sleeve 400.

Specifically, the supporting base 810 is further provided with a branch channel 816, and the branch channel 816 is in fluid communication with the liquid inlet hole 814, so that fluid (such as flushing liquid) flowing through the liquid inlet hole 814 can also flow into the inner cavity 101 of the housing 100 through the branch channel 816. Specifically, one end of the branch channel 816 is in communication with a gap between the first shaft sleeve 300 and the cavity bottom 812 of the mounting cavity 811, and the other end of the branch channel 816 is in communication with the inner cavity 101. In the embodiment shown in FIG. 32, the branch channel 816 is formed by partially recessing a cavity wall of the mounting cavity 811. In other words, in a normal state, the flushing liquid is divided into two streams after entering the mounting cavity 811 from the liquid inlet hole 814, one stream flows into the groove 310 of the first shaft sleeve 300 through the flushing liquid hole 320, and the other stream flows out through the branch channel 816. The branch channel 816 can ensure the flow of the flushing liquid when the sliding portion 220 blocks the flushing liquid hole 320.

In the embodiment shown in FIG. 32, two branch channels 816 are provided, and the two branch channels 816 are arranged opposite to each other. It should be understood that the number of the branch channel 816 can be adjusted according to design requirements, for example, in some embodiments, one or more than two branch channels 816 are provided.

In some embodiments, referring to FIGS. 24 to 26, the blocking member 500 is fixedly connected to the shaft portion 210 and is located between the first shaft sleeve 300 and the second shaft sleeve 400. The blocking member 500 can abut against the second shaft sleeve 400, so that the second shaft sleeve 400 can limit the range of movement of the shaft portion 210 in the direction approaching the second shaft sleeve 400. Specifically, the blocking member 500 may be a pushing stop ring 510.

In some embodiments, when the blocking member 500 abuts against the second shaft sleeve 400, the blocking member 500 does not seal the shaft hole 410 of the second shaft sleeve 400, so that the flushing liquid can flow into the shaft hole 410 of the second shaft sleeve 400 through a gap between the blocking member 500 and the second shaft sleeve 400. That is, fluid communication between the shaft hole 410 of the second shaft sleeve 400 and the inner cavity 101 is achieved. Specifically, an outer diameter of the pushing stop ring 510 is less than the outer diameters of the second rotor 620 and the second shaft sleeve 400, and the outer diameter of the pushing stop ring 510 is larger than the diameter of the shaft hole 410. The pushing stop ring 510 abuts against the second shaft sleeve 400, and the pushing stop ring 510 enables the second shaft sleeve 400 and the second rotor 620 spaced apart by a distance. Compared with the second rotor 620 directly being in contact with the second shaft sleeve 400, the pushing stop ring 510 can reduce a friction area between the second shaft sleeve 400 and the blocking member 500.

In other embodiments, the pushing stop ring 510 may also be formed by arranging a plurality of sector rings that are evenly spaced around the shaft portion 210 for one circumference, or may be understood as being formed by arranging a plurality of sector rings that are circumferentially discretely arranged.

Specifically, referring to FIG. 33, a surface of the blocking member 500 of the second shaft sleeve 400 is partially recessed to form a guiding groove 420, and the guiding groove 420 is in communication with the shaft hole 410 of the second shaft sleeve 400. When the blocking member 500 abuts against the second shaft sleeve 400, a part of the guiding groove 420 is not covered by the blocking member 500. For example, when the pushing stop ring 510 abuts against the second shaft sleeve 400, although the pushing stop ring 510 blocks the gap between the shaft hole 410 of the second shaft sleeve 400 and the shaft portion 210, the guiding groove 420 not covered by the pushing stop ring 510 can achieve fluid communication when the pushing stop ring 510 abuts against the second shaft sleeve 400, so as to ensure the smoothness of the flushing liquid. In addition, the guiding groove 420 is formed by partially recessing the surface of the second shaft sleeve 400 facing the blocking member 500, so that the flushing liquid can flow better between the blocking member 500 and the second shaft sleeve 400 to lubricate the contact surface between the blocking member 500 and the second shaft sleeve 400. The friction between the blocking member 500 and the second shaft sleeve 400 is reduced, the wear problem due to the friction between the blocking member 500 and the second shaft sleeve 400 is reduced, and the heat dissipation effect on the blocking member 500 and the second shaft sleeve 400 is achieved.

In some embodiments, referring to FIGS. 26 and 33, the second shaft sleeve 400 includes a first annular body 401 and a second annular body 402. A diameter of the first annular body 401 is less than that of the second annular body 402. A proximal end of the first annular body 401 is connected to a distal end of the second annular body 402. In the illustrated embodiment, the first annular body 401 and the second annular body 402 are integrally formed. The second annular body 402 is provided with a guiding groove 420. The housing 100 is provided with a mounting hole 102 adapted to the first annular body 401. The mounting hole 102 is located at the distal end of the housing 100. An inner wall of the housing 100 is provided with a limiting protrusion 103 surrounding the mounting hole 102. The first annular body 401 is mounted in the mounting hole 102. The second annular body 402 abuts against an end surface of a proximal end of the limiting protrusion 103, so as to position and mount the second shaft sleeve 400, and facilitate stable mounting of the second shaft sleeve 400.

In this embodiment, at least one of the first shaft sleeve 300, the second shaft sleeve 400, and the blocking member 500 is made of a ceramic. Compared with metal, ceramic has high machining accuracy, biocompatibility, high mechanical strength, and good wear resistance and corrosion resistance. In some embodiments, at least one of the first shaft sleeve 300 and the second shaft sleeve 400 has a sleeve body and a diamond coating provided on a surface of the sleeve body, so as to smooth the surfaces of the first shaft sleeve 300 and the second shaft sleeve 400 and improve wear resistance. At this time, the material of the shaft sleeve body may be a material having a certain rigidity, such as metal, ceramic, etc.

Specifically, a roughness of at least one of a hole wall of the shaft hole 410, a surface of the shaft portion 210, a surface of the sliding portion 220, and the groove wall of the groove 310 is less than or equal to 0.1 microns, thereby effectively reducing the frictional force between the shaft portion 210 and the hole wall of the shaft hole 410, and the frictional force between the sliding portion 220 and the groove wall of the groove 310.

Referring to FIG. 25, FIG. 26 and FIG. 34, the driving mechanism 10 further includes a first rotor 610 fixedly connected to the rotating shaft 200. The first rotor 610 is located between the first shaft sleeve 300 and the second shaft sleeve 400, and a gap is provided between the first rotor 610 and the first shaft sleeve 300, so as to avoid mutual wear between the first rotor 610 and the first shaft sleeve 300 and reduce the operation resistance of the driving mechanism 10. Specifically, the first rotor 610 is fixedly connected to the limiting surface 223 of the sliding portion 220. The limiting surface 223 can increase a connection area between the first rotor 610 and the rotating shaft 200, and improve the connection stability of the first rotor 610. The limiting surface 223 further serves to mount and position the first rotor 610 and to limit a distance that the first rotor 610 moves along the axis of the shaft portion 210 in the direction approaching the first shaft sleeve 300. Optionally, the first rotor 610 is fixed to the limiting surface 223 by adhering, welding, etc. The cylindrical surface 222 having a certain length can increase the distance between the first rotor 610 and the first shaft sleeve 300, so as to prevent the first rotor 610 from abutting against the first shaft sleeve 300 and causing the rotating shaft 200 to be stuck when the rotating shaft 200 swings.

The first rotor 610 includes a first flywheel 611 and a first magnet 612. The first flywheel 611 is fixedly connected to the rotating shaft 200. For example, the first flywheel 611 is fixedly connected to the limiting surface 223. The first magnet 612 is fixedly connected to the first flywheel 611. In some embodiments, the first magnet 612 is an annular Halbach array magnet. In the illustrated embodiment, the first rotor 610 is located in the inner cavity 101, and the first rotor 610 can rotate relative to the housing 100 and drive the rotating shaft 200 to rotate.

In some embodiments, referring to FIGS. 25 and 26, the driving mechanism 10 further includes a stator 700. The stator 700 and the first rotor 610 are arranged along the axis of the shaft portion 210, and the stator 700 is located between the first shaft sleeve 300 and the second shaft sleeve 400. Specifically, the stator 700 can generate a rotating magnetic field that drives the first magnet 612 of the first rotor 610 to rotate. By arranging the first rotor 610 and the stator 700 along the axis of the shaft portion 210, the overall diameter of the driving mechanism 10 can be reduced. In the illustrated embodiment, the stator 700 is fixedly mounted to the housing 100, the stator 700 is located in the inner cavity 101, and the shaft portion 210 rotatably extends through the stator 700. Optionally, the stator 700 is located between the first rotor 610 and the blocking member 500.

In some embodiments, the driving mechanism 10 further includes a second rotor 620 fixedly connected to the shaft portion 210, and the second rotor 620 is located between the first shaft sleeve 300 and the second shaft sleeve 400. In the illustrated embodiment, the blocking member 500 is located between the second rotor 620 and the second shaft sleeve 400. The blocking member 500 is fixedly connected to at least one of the second rotor 620 and the shaft portion 210, so that the blocking member 500, the rotating shaft 200, and the second rotor 620 rotate and move synchronously. In other words, the blocking member 500 may be directly fixed to only the second rotor 620, may be directly fixed to only the shaft portion 210, or may be directly fixed to both the second rotor 620 and the shaft portion 210.

In an embodiment, referring to FIGS. 24, 25, and 35, specially, the second rotor 620 includes a second flywheel 621 and a second magnet 622. The second flywheel 621 is fixedly connected to the shaft portion 210, and the second magnet 622 is fixedly connected to the second flywheel 621. In some embodiments, the second magnet 622 is an annular Halbach array magnet. It should be understood that the structure of the second flywheel 621 may be the same as that of the first flywheel 611, which will not be described herein again.

In the illustrated embodiment, the pushing stop ring 510 is an annular protrusion formed on a side of the second flywheel 621 away from the first rotor 610. The pushing stop ring 510 and the second flywheel 621 are integrally formed as a whole, which is convenient to mount and saves the adhering operation. In addition, the pushing stop ring 510 and the second rotor 620 may also be of separate structures prior to assembly. In this case, the pushing stop ring 510 may be fixed to at least one of the second rotor 620 or the shaft portion 210 by adhering or welding.

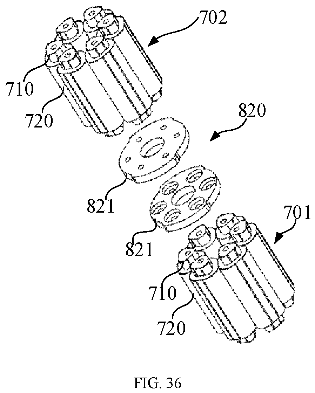

In some embodiments, referring to FIGS. 25, 26, and 36, the stator 700 includes a magnetic core 710 and a coil 720 wound around the magnetic core 710. The magnetic core 710 has a substantially columnar structure, that is, the magnetic core 710 does not have ahead portion (i.e., a pole shoe) having a large width. Compared with the magnetic core 710 having the pole shoe, the magnetic core 710 of the columnar structure can reduce the magnetic loss and increase the magnetic coupling density between the magnetic core 710 and the first magnet 612 and the second magnet 622 to increase the torque of the stator 700 to the first magnet 612 and the second magnet 622 (under the same current conditions). In addition, the magnetic core 710 without the head portion can also greatly reduce the problems of local magnetic short circuit and motor power reduction caused by the contact between the adjacent magnetic cores 710. Specifically, the magnetic core 710 extends in a direction consistent with the axial direction of the housing 100 or the axis of the shaft portion 210.

The first rotor 610, the stator 700, and the second rotor 620 are arranged sequentially along the axis of the shaft portion 210. In the illustrated embodiment, the stator 700 includes a first stator unit 701 and a second stator unit 702 arranged along the axis of the shaft portion 210. The first stator unit 701 and the second stator unit 702 each includes the magnetic core 710 and the coil 720. The first stator unit 701 can drive the first rotor 610 to rotate, and the second stator unit 702 can drive the second rotor 620 to rotate. Both the first stator unit 701 and the second stator unit 702 are fixedly accommodated in the inner cavity 101 of the housing 100. The shaft portion 210 rotatably extends through the first stator unit 701 and the second stator unit 702. The first rotor 610, the first stator unit 701, the second rotor 620, and the second stator unit 702 are arranged sequentially along the direction of the axis.

Specifically, the driving mechanism 10 further includes a magnetic conduction member 820 fixedly connected to the housing 100. The magnetic core 710 of the first stator unit 701 and the magnetic core 710 of the second stator unit 702 are both fixedly connected to the magnetic conduction member 820. The shaft portion 210 rotatably extends through the magnetic conduction member 820. The magnetic conduction member 820 serves to close a magnetic circuit, so as to promote and increase the generation of the magnetic flux and improve a coupling capability. Therefore, the magnetic conduction member 820 can function to close the magnetic circuit between the first stator unit 701 and the first rotor 610, and to close the magnetic circuit between the second stator unit 702 and the second rotor 620, so as to increase the magnetic flux, and further, the arrangement of the magnetic conduction member 820 is beneficial to reducing the overall diameter of the driving mechanism 10. In addition, the magnetic conduction member 820 can be directly fixedly connected to the housing 100 to position and mount of the first stator unit 701 and the second stator unit 702, thereby reducing the assembly difficulty of the first stator unit 701 and the second stator unit 702. Specifically, the magnetic conduction member 820 includes two magnetic conduction plates 821 that are stacked. One magnetic conduction plate 821 is fixedly connected to the magnetic core 710 of the first stator unit 701, and the other magnetic conduction plate 821 is fixedly connected to the magnetic core 710 of the second stator unit 702.

Since the driving device of this embodiment has a structure similar to that of the driving mechanism of the first embodiment, the driving mechanism of this embodiment and the blood pump having the driving mechanism of this embodiment also have effects similar to those of the first embodiment.

The foregoing embodiments are merely intended to describe the technical solutions of the present application, but not to limit the present application. Although the present application is described in detail with reference to the foregoing embodiments, those of ordinary skill in the art should understand that they may still make modifications to the technical solutions described in the foregoing embodiments or make equivalent replacements to some technical features thereof, without departing from the spirit and scope of the technical solutions of the embodiments of the present application, all of which fall within the protection scope of the present application.

## Claims

1. A driving mechanism configured to drive an impeller to rotate, the driving mechanism comprising:
a housing;
a rotating shaft rotatably mounted to the housing, the rotating shaft comprising a connecting end configured to be connected to the impeller and a spherical head end away from the connecting end;
a rotor fixedly connected to the rotating shaft;
a first shaft sleeve and a second shaft sleeve that are mounted to the housing, wherein the first shaft sleeve is provided with a groove having a concave spherical wall, the rotating shaft rotatably extends through the second shaft sleeve, the spherical head end is movably provided in the groove and is capable of abutting against the spherical wall, and the rotor is located between the first shaft sleeve and the second shaft sleeve; and
a blocking member fixedly connected to at least one of the rotating shaft and the rotor, wherein the blocking member is located between the rotor and the second shaft sleeve, and the blocking member is capable of abutting against the second shaft sleeve.

2. The driving mechanism according to claim 1, wherein the groove is provided with a notch, the rotating shaft extends through the notch, and the notch of the groove is rounded;
and/or a length of the spherical head end in an axial direction of the rotating shaft is less than a depth of the groove.

3. The driving mechanism according to claim 1, wherein the first shaft sleeve is further provided with a liquid feeding hole in communication with the groove, an opening of the liquid feeding hole is located on the spherical wall;
wherein and the opening is located at a center of the spherical wall;
and/or a diameter of the opening is 1/9 to 1/3 of a diameter of a sphere where the spherical head end is located.

4. The driving mechanism according to claim 1, wherein the first shaft sleeve is further provided with a liquid feeding hole in communication with the groove, the driving mechanism further comprises a supporting base fixedly connected to the housing, the supporting base is provided with a mounting cavity and a liquid inlet hole in communication with the mounting cavity, the first shaft sleeve is mounted in the mounting cavity, and the liquid feeding hole is in fluid communication with the liquid inlet hole.

5. The driving mechanism according to claim 4, wherein the mounting cavity comprises a cavity bottom, an opening of the liquid inlet hole is located at the cavity bottom, a supporting step is provided in the mounting cavity, and the supporting step abuts against the first shaft sleeve, so that the first shaft sleeve is spaced apart from the cavity bottom by a certain distance;
and/or the supporting base is further provided with a branch channel in communication with the liquid inlet hole, so that fluid entering the liquid inlet hole is further capable of flowing into the housing through the branch channel.

6. The driving mechanism according to claim 1, wherein the second shaft sleeve has a shaft hole and a stopping surface perpendicular to a central axis of the shaft hole, the rotating shaft rotatably extends through the shaft hole, the blocking member comprises a blocking surface perpendicular to an axis of the rotating shaft, the blocking surface is opposite to the stopping surface, and the blocking surface is capable of abutting against the stopping surface.

7. The driving mechanism according to claim 6, wherein a roughness of at least one of the blocking surface and the stopping surface is less than or equal to 0.1 microns,
or at least one of the blocking surface and the stopping surface is a ceramic surface,
or the blocking surface is made of diamond.

8. The driving mechanism according to claim 1, wherein a separating ring is arranged in the housing, the separating ring divides an inner cavity of the housing into a limiting cavity and an accommodating cavity, the limiting cavity and the accommodating cavity are arranged along an axis of the rotating shaft, the second shaft sleeve is accommodated in the limiting cavity and abuts against the separating ring, the rotor is accommodated in the accommodating cavity, so that the separating ring is located between the second shaft sleeve and the rotor;
wherein when the blocking member abuts against the second shaft sleeve, the blocking member is at least partially located in an inner ring of the separating ring, a gap for fluid to flow is formed between the blocking member and a wall of the inner ring of the separating ring, and the separating ring is spaced apart from the rotor by a distance.

9. The driving mechanism according to claim 8, wherein the second shaft sleeve is provided with a shaft hole, the rotating shaft rotatably extends through the shaft hole, a gap for the fluid to flow is formed between the rotating shaft and a hole wall of the shaft hole, a surface of the second shaft sleeve facing the blocking member is partially recessed to form a guiding groove in communication with the shaft hole, when the blocking member abuts against the second shaft sleeve, part of the guiding groove is not covered by the blocking member.

10. The driving mechanism according to claim 1, wherein the rotor comprises a first rotor and a second rotor that are arranged along an axis of the rotating shaft, both the first rotor and the second rotor are fixedly connected to the rotating shaft), both the first rotor and the second rotor are located between the first shaft sleeve and the second shaft sleeve, and the blocking member is located between the second rotor and the second shaft sleeve;
wherein the driving mechanism further comprises a stator capable of driving the rotor to rotate,the stator comprises a first stator unit and a second stator unit that are arranged along the axis of the rotating shaft, both the first stator unit and the second stator unit are located between the first rotor and the second rotor, the first stator unit is capable of driving the first rotor to rotate, the second stator unit is capable of driving the second rotor to rotate, and the first stator unit and the second stator unit each comprises a magnetic core and a coil wound around the magnetic core;
wherein the driving mechanism further comprises a magnetic conduction member fixedly connected to the housing, both the magnetic core of the first stator unit and the magnetic core of the second stator unit are fixedly connected to the magnetic conduction member, and the rotating shaft rotatably extends through the first stator unit, the second stator unit, and the magnetic conduction member.

11. The driving mechanism according to claim 1, wherein the rotating shaft comprises a shaft portion and a sliding portion provided at an end of the shaft portion, the shaft portion rotatably extends through the second shaft sleeve, an end of the shaft portion away from the sliding portion is the connecting end, the sliding portion is the spherical head end, and the sliding portion comprises a spherical crown surface;
wherein a depth of the groove is less than or equal to a height of the spherical crown surface in an axial direction of the shaft portion, the sliding portion is movably provided in the groove, the spherical crown surface slidably abuts against the spherical wall of the groove, and the blocking member is fixedly connected to the shaft portion.

12. The driving mechanism according to claim 11, wherein the spherical crown surface slidably abuts against the spherical wall, a radius of a sphere where the spherical wall is located is greater than a radius of a sphere where the spherical crown surface is located, a diameter of the groove gradually increases along an axis of the first shaft sleeve and in a direction approaching the second shaft sleeve.

13. The driving mechanism according to claim 11, wherein the depth of the groove is greater than or equal to a half of a height of the spherical crown surface in the axial direction of the shaft portion;
and/or the groove is provided with a notch, an edge of the notch is rounded, and the sliding portion extends through the notch of the groove.

14. The driving mechanism according to claim 11, wherein the spherical crown surface slidably abuts against the spherical wall, and a difference between a radius of a sphere where the spherical wall is located and a radius of a sphere wherein the spherical crown surface is located is defined as D, and 0.04 mm ≤ D ≤ 0.06 mm;
and/or the spherical crown surface slidably abuts against the spherical wall, and the depth of the groove is 0.6 to 1 times of the radius of the sphere where the spherical wall is located.

15. The driving mechanism according to claim 11, wherein the rotor comprises a first rotor and a second rotor, the first rotor is fixedly connected to the shaft portion, the first rotor is located between the first shaft sleeve and the second shaft sleeve, a gap is formed between the first rotor and the first shaft sleeve, the second rotor is located between the first shaft sleeve and the blocking member, and at least one of the blocking member and the second shaft sleeve is made of ceramic.

16. The driving mechanism according to claim 15, wherein the sliding portion further comprises a cylindrical surface and a limiting surface, one end of the cylindrical surface is connected to the spherical crown surface, another end of the cylindrical surface is connected to the limiting surface, an axis of the cylindrical surface coincides with an axis of the shaft portion, the limiting surface is perpendicular to the axis of the shaft portion, and the first rotor abuts against the limiting surface.

17. A blood pump, comprising an impeller and a driving mechanism, the driving mechanism comprising:
a housing;
a rotating shaft rotatably mounted to the housing, the rotating shaft comprising a connecting end configured to be connected to the impeller and a spherical head end away from the connecting end;
a rotor fixedly connected to the rotating shaft;
a first shaft sleeve and a second shaft sleeve that are mounted to the housing, wherein the first shaft sleeve is provided with a groove having a concave spherical wall, the rotating shaft rotatably extends through the second shaft sleeve, the spherical head end is movably provided in the groove and is capable of abutting against the spherical wall, and the rotor is located between the first shaft sleeve and the second shaft sleeve; and
a blocking member fixedly connected to at least one of the rotating shaft and the rotor, wherein the blocking member is located between the rotor and the second shaft sleeve, and the blocking member is capable of abutting against the second shaft sleeve;
wherein the impeller is connected to the connecting end of the rotating shaft and is capable of rotating along with the rotating shaft.

18. The blood pump according to claim 17, further comprising a cannula assembly connected to the driving mechanism, wherein the cannula assembly comprises an inserting tube, a connecting tube sleeved on the inserting tube, and an outlet tube sleeved in the connecting tube, an inner wall of an end of the connecting tube is connected to an outer wall of the inserting tube, the outlet tube comprises a connecting portion and an outlet portion away from the connecting tube, an outer wall of the connecting portion is connected to an inner wall of an end of the connecting tube away from the inserting tube, and the impeller is rotatably provided in the outlet tube.

19. The blood pump according to claim 17, wherein an inner wall of the connecting tube is provided with a limiting protruding ring, the limiting protruding ring is in an annular shape protruding from the inner wall of the connecting tube, the limiting protruding ring has a first end surface and a second end surface that are arranged along an axial direction of the connecting tube, the first end surface abuts against an end portion of the inserting tube, and the second end surface abuts against an end portion of the connecting portion.

20. The driving mechanism according to claim 19, wherein the first end surface protrudes more from the inner wall of the connecting tube in a radial direction than the second end surface, an edge of the first end surface and an edge of the second end surface are connected by a transition surface, the edge of the first end surface is coplanar with an inner wall of the inserting tube, the edge of the second end surface is coplanar with an inner wall of the outlet tube, and the transition surface is provided as one of an outer convex curved surface, an inner concave curved surface or an inclined surface.
